# EUROPEAN PATENT APPLICATION

(11) **EP 4 563 686 A1**
(43) Date of publication of application: **04.06.2025**
(21) Application number: 23846070.3
(22) Date of filing: 19.06.2023
(51) Int. Cl.: C12M 1/00, C07K 1/16, C07K 1/34, C12M 1/12, C12N 5/071, C12N 15/10

(54) **PURIFYING AND CONCENTRATING DEVICE FOR LIQUID COMPRISING MINUTE USEFUL SUBSTANCE AND METHOD FOR PRODUCING PURIFIED AND CONCENTRATED LIQUID OF MINUTE USEFUL SUBSTANCE USING SAME**

(30) Priority: 28.07.2022 JP 2022120666
(71) Applicant: Daicel Corporation, Osaka-shi, Osaka 530-0011 (JP)
(72) Inventor: SHOJI, Koichi, Tokyo 108-8230 (JP); SATO, Hiroshi, Tokyo 108-8230 (JP); NAKATSUKA, Shuji, Tokyo 108-8230 (JP); UCHIMURA, Seiichi, Tokyo 108-8230 (JP)
(74) Representative: Grünecker Patent- und Rechtsanwälte PartG mbB
(86) International application number: PCT/JP2023/022644
(87) International publication number: WO 2024/024336

(57) **Abstract**

There are provided a purifying and concentrating apparatus for purifying and concentrating a liquid containing a minute useful substance, which can produce a liquid containing a minute useful substance with high purity and high concentration (purified and concentrated liquid of the minute useful substance) and can more efficiently prepare the purified and concentrated liquid, and a method for producing a purified and concentrated liquid of a minute useful substance using the same. A purifying and concentrating apparatus for purifying and concentrating a liquid containing a minute useful substance, the purifying and concentrating apparatus including: a raw material supply unit configured to supply a liquid containing a minute useful substance; a removing unit configured to remove an impurity from the liquid; a purifying and concentrating unit configured to purify and concentrate, with a hollow fiber membrane, the liquid supplied from the raw material supply unit or the removing unit; and a first control unit configured to control a timing of flow of the liquid from the raw material supply unit to the purifying and concentrating unit or flow of the liquid from the removing unit to the purifying and concentrating unit.

## Description

### Technical Field

The present disclosure relates to a purifying and concentrating apparatus for purifying and concentrating a liquid containing a minute useful substance and a method for producing a purified and concentrated liquid of a minute useful substance using the same.

### Background Art

As a method for separating and purifying a minute useful substance from a liquid containing the minute useful substance such as an extracellular vesicle, an antibody, a virus, a protein, or a nucleic acid, for example, a method using a separation membrane is known.

Patent Document 1 describes a method for recovering useful substances. The method includes a bleeding process of discharging a culture solution from a cell culture tank and adding a fresh culture medium in the same amount as the discharged culture solution to the culture tank, and a filtration process of filtering the culture solution extracted from the culture tank using a porous membrane having substantially no dense layer. The filtration in the filtration process is tangential flow filtration, and a velocity of a permeated liquid in the filtration process is 1.0 LMH or less. Patent Document 1 also describes that the minute useful substance may be selected from the group consisting of proteins, viruses, exosomes, and nucleic acids.

Patent Document 2 describes an exosome extraction apparatus and an exosome extraction method that can efficiently collect a large amount of exosomes, which are one type of extracellular vesicles. In the exosome extraction apparatus described in Patent Document 2, a raw material liquid containing exosomes supplied from a raw material supply unit is fed to a first storage unit, and is then circulated from the first storage unit through a circulation path including a first filter and a second filter having different roles to concentrate the exosomes in the liquid.

Patent Document 1: JP 2018-76291 A
Patent Document 2: JP 2021-145649 A

### Summary of Invention

In general, an impurity is often contained in a raw material liquid containing a minute useful substance. Thus, there is a demand for a purifying and concentrating apparatus capable of preparing a liquid containing a minute useful substance with high purity and high concentration (a purified and concentrated liquid of the minute useful substance). Furthermore, there is also a demand for a purifying and concentrating apparatus capable of more efficiently preparing the purified and concentrated liquid.

The present invention has been made in view of the above circumstances, and is directed to providing a purifying and concentrating apparatus for purifying and concentrating a liquid containing a minute useful substance, which is capable of producing a liquid containing a minute useful substance with high purity and high concentration (a purified and concentrated liquid of a minute useful substance) and more efficiently preparing the purified and concentrated liquid, and a method for producing a purified and concentrated liquid of a minute useful substance using the apparatus.

To solve the problems described above, the present disclosure can adopt the following means.
[1] A purifying and concentrating apparatus for purifying and concentrating a liquid containing a minute useful substance, the purifying and concentrating apparatus including:
   a raw material supply unit configured to supply a liquid containing a minute useful substance;
   a removing unit configured to remove an impurity from the liquid;
   a purifying and concentrating unit configured to purify and concentrate, with a hollow fiber membrane, the liquid supplied from the raw material supply unit or the removing unit; and
   a first control unit configured to control a timing of flow of the liquid from the raw material supply unit to the purifying and concentrating unit or flow of the liquid from the removing unit to the purifying and concentrating unit.
[2] The purifying and concentrating apparatus according to [1], in which the removing unit includes a carrier column configured to remove the impurity from the liquid, and the carrier column is connected to a flow path through which the liquid from which the impurity has been removed is supplied to the purifying and concentrating unit.
[3] The purifying and concentrating apparatus according to [1] or [2],
   in which
   the removing unit includes a filtration member that removes the impurity from the liquid, and
   the filtration member is interposed in the flow path through which the liquid is supplied from the raw material supply unit to the removing unit.
[4] The purifying and concentrating apparatus according to [2] or [3], in which
   the removing unit includes a first tank that stores the liquid treated by the carrier column,
   the first tank is disposed in a flow path through which the liquid is supplied from the carrier column to the purifying and concentrating unit, and the first tank is connected to a flow path through which the liquid in the first tank is supplied to the purifying and concentrating unit, and
   the first control unit controls a timing at which the liquid in the first tank is supplied to the purifying and concentrating unit.
[5] The purifying and concentrating apparatus according to any one of [1] to [4], in which the hollow fiber membrane has an average inner diameter of 0.2 to 1.4 mm and a molecular weight cutoff of 10000 to 1000000.
[6] The purifying and concentrating apparatus according to any one of [1] to [5], in which the purifying and concentrating unit includes a hollow fiber membrane module in which a plurality of the hollow fiber membranes are accommodated in a container having an inlet/outlet for the liquid, and the container has at least a first liquid inlet/outlet and a second liquid inlet/outlet for supplying the liquid to the hollow fiber membranes, and a third liquid inlet/outlet for discharging the liquid having permeated through the hollow fiber membranes.
[7] The purifying and concentrating apparatus according to [6], in which the hollow fiber membrane module is of an internal pressure type.
[8] The purifying and concentrating apparatus according to [6] or [7], in which the purifying and concentrating unit includes:
   a second tank that stores the liquid supplied from the raw material supply unit or the removing unit, the second tank being connected to a flow path through which the liquid is supplied to the first liquid inlet/outlet of the hollow fiber membrane module;
   a third tank that stores the liquid supplied from the raw material supply unit or the removing unit, the third tank being connected to a flow path through which the liquid is supplied to the second liquid inlet/outlet of the hollow fiber membrane module;
   a pressurizing device configured to selectively pressurize either the second tank or the third tank; and
   a second control unit configured to perform control in such a manner that the pressurizing device selectively pressurizes an inside of the second tank or the third tank to alternately supply the liquid in the second tank and the liquid in the third tank to the hollow fiber membrane module.
[9] The purifying and concentrating apparatus according to [8], wherein the second control unit sets values for a liquid amount in the second tank and a liquid amount in the third tank, and causes the pressurizing device to alternately pressurize the second tank and the third tank in such a manner that the liquid amount in the second tank and the liquid amount in the third tank are the set values.
[10] The purifying and concentrating apparatus according to [9],
   in which
   each of the second tank and the third tank includes an in-tank liquid amount monitoring device including an optical sensor at an inlet/outlet of the liquid at a tank bottom portion, the in-tank liquid amount monitoring device is configured to, by the optical sensor, irradiate an inside of the inlet/outlet of the liquid with light to detect a transmittance of the light and monitor the liquid amount in the corresponding tank based on a change in the transmittance, and
   the second control unit determines the liquid amount in the second tank and the liquid amount in the third tank by the in-tank liquid amount monitoring device to determine a tank to be pressurized by the pressurizing device.
[11] The purifying and concentrating apparatus according to [9] or [10],
   in which
   the purifying and concentrating unit includes a camera that monitors a liquid level position in the second tank and a liquid level position in the third tank, and
   the second control unit determines the tank to be pressurized by the pressurizing device based on information on the liquid level position in the second tank and the liquid level position in the third tank output from the camera.
[12] The purifying and concentrating apparatus according to any one of [8] to [11],
   in which
   the purifying and concentrating unit includes:
   a fourth tank that stores a diluent, the fourth tank being connected to a flow path through which the diluent is supplied to the third liquid inlet/outlet of the hollow fiber membrane module; and
   a third control unit configured to control a timing at which the diluent in the fourth tank is supplied to the third liquid inlet/outlet of the hollow fiber membrane module, and
   the third control unit supplies the diluent in the fourth tank from the third liquid inlet/outlet of the hollow fiber membrane module in a case where a total of the liquid amount in the second tank and the liquid amount in the third tank is equal to or less than a value set by the second control unit.
[13] The purifying and concentrating apparatus according to any one of [8] to [12],
   in which
   the purifying and concentrating unit includes:
   a fifth tank connected to the first liquid inlet/outlet or the second liquid inlet/outlet of the hollow fiber membrane module by a flow path, the fifth tank recovering the liquid in the second tank and the liquid in the third tank; and
   a fourth control unit configured to control recovery of the liquid in the second tank and the liquid in the third tank to the fifth tank.
[14] The purifying and concentrating apparatus according to [13],
   in which
   each of the second tank and the third tank includes an in-tank liquid amount monitoring device including an optical sensor at an inlet/outlet of the liquid at a tank bottom portion, and the in-tank liquid amount monitoring device is configured to, by the optical sensor, irradiate an inside of the inlet/outlet of the liquid with light to detect a transmittance of the light and monitor a liquid amount in the corresponding tank based on a change in the transmittance, and
   the fourth control unit causes the pressurizing device to selectively pressurize the inside of the second tank or the inside of the third tank to recover the liquid in the second tank and the liquid in the third tank to the fifth tank, and determines a liquid amount in the second tank and a liquid amount in the third tank by the in-tank liquid amount monitoring device to stop the pressurizing device.
[15] The purifying and concentrating apparatus according to [13] or [14],
   in which
   the purifying and concentrating unit includes a camera that monitors a liquid level position in the second tank and a liquid level position in the third tank, and
   the fourth control unit causes the pressurizing device to selectively pressurize the inside of the second tank or the inside of the third tank to recover the liquid in the second tank and the liquid in the third tank to the fifth tank, and stops the pressurizing device based on information on the liquid level position in the second tank and the liquid level position in the third tank output from the camera.
[16] The purifying and concentrating apparatus according to any one of [1] to [15], further including a management unit,
   in which
   the purifying and concentrating apparatus has a configuration in which elements including the raw material supply unit, the removing unit, and the purifying and concentrating unit are disposed in a housing, and
   the management unit is configured to manage a degree of cleanliness in the housing, and
   the management unit includes a fan filter device that supplies filtered air into the housing.
[17] The purifying and concentrating apparatus according to [16], in which the management unit further includes a UV lamp.
[18] The purifying and concentrating apparatus according to any one of [2] to [17], in which the removing unit includes a sixth tank that stores a cleaning liquid for cleaning the carrier column and a seventh tank that stores an equilibrating liquid for equilibrating the carrier column.
[19] The purifying and concentrating apparatus according to any one of [3] to [18], in which the filtration member is disposed below the raw material supply unit, and the liquid is supplied from the raw material supply unit to the filtration member by an own weight of the liquid.
[20] The purifying and concentrating apparatus according to any one of [8] to [19], in which side surfaces of the second tank and the third tank in a longitudinal direction thereof are arranged in parallel with a longitudinal direction of the hollow fiber membrane module.
[21] The purifying and concentrating apparatus according to [20],
   in which the removing unit includes:
   a carrier column that removes an impurity from the liquid, the carrier column being connected to a flow path through which the liquid from which the impurity has been removed is supplied to the purifying and concentrating unit; and
   a first tank that stores the liquid treated by the carrier column, and
   the second tank and the third tank are connected to the carrier column and the first tank by a flow path, and are disposed at positions above the carrier column and the first tank.
[22] The purifying and concentrating apparatus according to any one of [1] to [21], in which the minute useful substance is selected from the group consisting of an extracellular vesicle, an antibody, a virus, a protein, an enzyme, and a nucleic acid.
[23] A method for producing the liquid in which a minute useful substance is purified and concentrated using the purifying and concentrating apparatus described in any one of [1] to [22], the method including
   supplying a raw material liquid containing the minute useful substance to the purifying and concentrating apparatus to remove an impurity from the liquid, and/or
   purifying and concentrating the liquid with a hollow fiber membrane.
[24] The method for producing a purified and concentrated liquid of a minute useful substance according to [23], in which the removing the impurity from the liquid includes adsorbing and removing the impurity from the liquid by a carrier column.
[25] The method for producing a purified and concentrated liquid of a minute useful substance according to [23] or [24], in which the purifying and concentrating the liquid with the hollow fiber membrane includes subjecting the liquid to alternating tangential flow filtration with a hollow fiber membrane module.
[26] The method for producing a purified and concentrated liquid of a minute useful substance according to any one of [23] to [25], in which the purifying and concentrating the liquid with the hollow fiber membrane includes subjecting the liquid to alternating tangential flow filtration with a hollow fiber membrane module while recovering the minute useful substance deposited on a surface of the hollow fiber membrane.
[27] The method for producing a purified and concentrated liquid of a minute useful substance according to any one of [23] to [26],
   in which
   purifying and concentrating the liquid with the hollow fiber membrane includes supplying a diluent to the liquid concentrated to further purify and concentrate the liquid, and
   the diluent is supplied to the liquid in a case where an amount (B) of the concentrated liquid relative to an amount (A) of the raw material liquid (B/A) is from 1/2 to 1/200.
[28] The method for producing a purified and concentrated liquid of a minute useful substance according to any one of [24] to [27], in which the removing the impurity from the liquid by the carrier column and the purifying and concentrating the liquid with the hollow fiber membrane are performed in parallel.
[29] The method for producing a purified and concentrated liquid of a minute useful substance according to any one of [23] to [28], in which the minute useful substance is at least one selected from the group consisting of an extracellular vesicle, an antibody, a virus, a protein, an enzyme, and a nucleic acid.
[30-1] A purifying and concentrating apparatus for purifying and concentrating a liquid containing a minute useful substance, the purifying and concentrating apparatus including:
   a raw material supply unit configured to supply a liquid containing a minute useful substance; and a purifying and concentrating unit configured to purify and concentrate, with a hollow fiber membrane module, the liquid supplied from the raw material supply unit,
   in which
   the hollow fiber membrane module has a first liquid inlet/outlet and a second liquid inlet/outlet for supplying the liquid to a hollow fiber membrane, and a third liquid inlet/outlet for discharging the liquid having permeated through the hollow fiber membrane,
   the purifying and concentrating unit includes
   a second tank that stores the liquid supplied from the raw material supply unit, the second tank being connected to a flow path through which the liquid is supplied to the first liquid inlet/outlet of the hollow fiber membrane module,
   a third tank that stores the liquid supplied from the raw material supply unit, the third tank being connected to a flow path through which the liquid is supplied to the second liquid inlet/outlet of the hollow fiber membrane module,
   a pressurizing device configured to selectively pressurize either the second tank or the third tank,
   a second control unit configured to perform control in such a manner that the pressurizing device selectively pressurizes an inside of the second tank and an inside of the third tank to alternately supply the liquid in the second tank and the liquid in the third tank to the hollow fiber membrane module, and
   a monitoring device configured to monitor an amount of the liquid in the second tank and an amount of the liquid the third tank,
   the second control unit sets values for a liquid amount in the second tank and a liquid amount in the third tank, and causes the pressurizing device to alternately pressurize the second tank and the third tank in such a manner that the liquid amount in the second tank and the liquid amount in the third tank are the set values, and the liquid amount in the second tank and the liquid amount in the third tank are determined based on information from the monitoring device.
[30-2] A purifying and concentrating apparatus for purifying and concentrating a liquid containing a minute useful substance, the purifying and concentrating apparatus including:
   a raw material supply unit configured to supply a liquid containing a minute useful substance; and
   a purifying and concentrating unit configured to purify and concentrate, with a hollow fiber membrane module, the liquid supplied from the raw material supply unit,
   in which
   the hollow fiber membrane module has a first liquid inlet/outlet and a second liquid inlet/outlet for supplying the liquid to a hollow fiber membrane, and a third liquid inlet/outlet for discharging the liquid having permeated through the hollow fiber membrane,
   the purifying and concentrating unit includes
   a second tank that stores the liquid supplied from the raw material supply unit, the second tank being connected to a flow path through which the liquid is supplied to the first liquid inlet/outlet of the hollow fiber membrane module,
   a third tank that stores the liquid supplied from the raw material supply unit, the third tank being connected to a flow path through which the liquid is supplied to the second liquid inlet/outlet of the hollow fiber membrane module,
   a liquid feeder configured to selectively feed the liquid in the second tank or the liquid in the third tank to the hollow fiber membrane module,
   a second control unit configured to perform control in such a manner that the liquid feeder selectively supplies the liquid in the second tank or the liquid in the third tank to the hollow fiber membrane module, and
   a monitoring device configured to monitor an amount of the liquid in the second tank and an amount of the liquid in the third tank,
   the second control unit sets values for a liquid amount in the second tank and a liquid amount in the third tank, and performs control in such a manner that the liquid feeder selectively feeds the liquid from the second tank or the third tank to the hollow fiber membrane module in a case where the liquid amount in the second tank and the liquid amount in the third tank are the set values, and
   the liquid amount in the second tank and the liquid amount in the third tank are determined based on information from the monitoring device.
[31] The purifying and concentrating apparatus according to [30-1] or [30-2], in which the hollow fiber membrane has an average inner diameter of 0.2 to 2.0 mm and a molecular weight cutoff of 5000 to 3000000.
[32] The purifying and concentrating apparatus according to any one of [30-1] to [31], in which the monitoring device includes a camera that monitors a liquid level position in the second tank and a liquid level position in the third tank.
[33] The purifying and concentrating apparatus according to any one of [30-1] to [32], in which the monitoring device includes optical sensors each provided at an inlet/outlet of the liquid at a bottom portion of each of the second tank and the third tank, and configured to irradiate an inside of the inlet/outlet of the liquid with light to detect a transmittance of the light, and monitor an amount of the liquid in the corresponding tank based on a change in the transmittance.
[34] The purifying and concentrating apparatus according to any one of [30-1] to [33], in which the hollow fiber membrane module is of an internal pressure type.
[35] The purifying and concentrating apparatus according to any one of [30-1] to [34],
   in which
   the purifying and concentrating unit includes:
   a fourth tank that stores a diluent, the fourth tank being connected to a flow path through which the diluent is supplied to the third liquid inlet/outlet of the hollow fiber membrane module; and
   a third control unit configured to control a timing at which the diluent in the fourth tank is supplied to the third liquid inlet/outlet of the hollow fiber membrane module, and
   the third control unit supplies the diluent in the fourth tank from the third liquid inlet/outlet of the hollow fiber membrane module in a case where a total of the liquid amount in the second tank and the liquid amount in the third tank is equal to or less than a value set by the first control unit.
[36] The purifying and concentrating apparatus according to any one of [30-1] to [35],
   in which
   the purifying and concentrating unit includes:
   a fifth tank connected to the first liquid inlet/outlet or the second liquid inlet/outlet of the hollow fiber membrane module by a flow path, the fifth tank recovering the liquid in the second tank and the liquid in the third tank; and
   a fourth control unit configured to control recovery of the liquid to the fifth tank.
[37] The purifying and concentrating apparatus according to [36], in which the fourth control unit causes the pressurizing device to selectively pressurize an inside of the second tank or an inside of the third tank to recover the liquid in the second tank and the liquid in the third tank to the fifth tank, determines the liquid amount in the second tank and the liquid amount in the third tank by monitoring device, and causes the pressurizing device to stop.
[38] The purifying and concentrating apparatus according to any one of [30-1] to [37], in which the minute useful substance is selected from the group consisting of an electroactive polymer, a rare metal colloidal particle, a pigment, a dye, an inorganic or organic nanoparticle, a protein, a hormone, a cytokine, a proliferative factor, an angiogenic factor, a growth factor, an enzyme, an antibody, a plasma protein, a virus, an extracellular vesicle, a fermentum, a yeast, a cell, polysaccharides, and microalgae.
[39] The purifying and concentrating apparatus according to any one of [1] to [38], in which a biomolecule of a type different from the minute useful substance is attached to at least a part of the hollow fiber membrane.
[40] The purifying and concentrating apparatus according to any one of [12] to [22] or any one of [35] to [39], in which
   the hollow fiber membrane module has at least two third liquid inlets/outlets, and
   the third control unit supplies the diluent in the fourth tank from one of the at least two third liquid inlets/outlets of the hollow fiber membrane module in a state where flow of the liquid through the first liquid inlet/outlet and the second liquid inlet/outlet of the hollow fiber membrane module is stopped, and then supplies the diluent in the fourth tank from at least one of the at least two third liquid inlets/outlets of the hollow fiber membrane module in a state where the flow of the liquid through the first liquid inlet/outlet and the second liquid inlet/outlet is enabled.
[41] A method for producing a purified and concentrated liquid of a minute useful substance, the method including:
   removing an impurity from a raw material liquid containing a minute useful substance and the impurity; and then,
   purifying and concentrating the liquid,
   in which
   the purifying and concentrating the liquid includes purifying and concentrating the liquid using a hollow fiber membrane module, and in a process of the purifying and concentrating, a diluent is injected under pressure from a permeated liquid side of the hollow fiber membrane module to subject the hollow fiber membrane to backwashing treatment.
[42] The method for producing a purified and concentrated liquid of a minute useful substance according to [41], in which the hollow fiber membrane accommodated in the hollow fiber membrane module is made of a resin material containing cellulose acetate.
[43] The method for producing a purified and concentrated liquid of a minute useful substance according to [42], in which the hollow fiber membrane accommodated in the hollow fiber membrane module has a molecular weight cutoff of 100000 to 1000000.
[44] The method for producing a purified and concentrated liquid of a minute useful substance according to any one of [41] to [43], in which the removing the impurity from the liquid includes adsorbing and removing the impurity from the liquid by a carrier column.
[45] The method for producing a purified and concentrated liquid of a minute useful substance according to any one of [41] to [44], in which the purifying and concentrating the liquid using the hollow fiber membrane module includes subjecting the liquid to alternating tangential flow filtration.
[46] The method for producing a purified and concentrated liquid of a minute useful substance according to any one of [41] to [45], in which the purifying and concentrating the liquid using the hollow fiber membrane module includes supplying a diluent to the purified and concentrated liquid to further purify and concentrate the liquid, and the diluent is supplied to the liquid in a case where an amount (B) of the purified and concentrated liquid relative to an amount (A) of the raw material liquid (B/A) is from 1/2 to 1/200.
[47] The method for producing a purified and concentrated liquid of a minute useful substance according to any one of [44] to [46], in which the removing the impurity from the liquid by the carrier column and the purifying and concentrating the liquid using the hollow fiber membrane module are performed in parallel.
[48] The method for producing a purified and concentrated liquid of a minute useful substance according to any one of [41] to [47], in which the minute useful substance is at least one selected from the group consisting of an extracellular vesicle, an antibody, a virus, a protein, an enzyme, and a nucleic acid.
[49] The method for producing a purified and concentrated liquid of a minute useful substance according to any one of [41] to [48], in which a biomolecule of a type different from the minute useful substance is attached to at least a part of the hollow fiber membrane.
[50] The method for producing a purified and concentrated liquid of a minute useful substance according to any one of [41] to [49], further including subjecting the hollow fiber membrane module to flushing treatment prior to the backwashing treatment.

According to the present disclosure, it is possible to provide a purifying and concentrating apparatus for purifying and concentrating a liquid containing a minute useful substance, which can produce a liquid containing a minute useful substance with high purity and high concentration (a purified and concentrated liquid of a minute useful substance) and can more efficiently prepare the purified and concentrated liquid, and a method for producing a purified and concentrated liquid of a minute useful substance using the same. The purifying and concentrating apparatus according to an embodiment of the present disclosure is also useful as an apparatus capable of purifying and concentrating a minute useful substance by automatic control.

### Brief Description of Drawings

FIG. 1 is a schematic configuration diagram illustrating an example of a purifying and concentrating apparatus according to a first embodiment.
FIG. 2 is a schematic configuration diagram illustrating an example of a removing unit according to the first embodiment.
FIG. 3 is a schematic configuration diagram illustrating an example of the removing unit according to the first embodiment.
FIG. 4 is a schematic configuration diagram illustrating an example of the removing unit according to the first embodiment.
FIG. 5 is a schematic configuration diagram illustrating an example of the removing unit according to the first embodiment.
FIG. 6 is a schematic configuration diagram illustrating an example of the removing unit according to the first embodiment.
FIG. 7 is a schematic cross-sectional view illustrating an example of a hollow fiber membrane module in the purifying and concentrating apparatus of the present disclosure.
FIG. 8 is a schematic configuration diagram illustrating an example of a purifying and concentrating unit according to the first embodiment.
FIG. 9 is a schematic configuration diagram illustrating an example of the purifying and concentrating unit according to the first embodiment.
FIG. 10 is a schematic configuration diagram illustrating an example of the purifying and concentrating unit according to the first embodiment.
FIG. 11 is a schematic configuration diagram illustrating an example of the purifying and concentrating unit according to the first embodiment.
FIG. 12 is a schematic configuration diagram illustrating an example of the purifying and concentrating apparatus according to the first embodiment.
FIG. 13 is a schematic configuration diagram illustrating an example of an overall configuration of the purifying and concentrating apparatus according to the first embodiment.
FIG. 14 is a schematic configuration diagram illustrating an example of an overall configuration of a purifying and concentrating apparatus according to a second embodiment.
FIG. 15 is an example of a device for measuring γ-globulin permeability of a hollow fiber membrane.
FIG. 16 is a flowchart illustrating an example of purifying and concentrating treatment using the purifying and concentrating apparatus according to the first embodiment.
FIG. 17 is a graph showing results of measuring concentrated liquids prepared in Example 4 and Comparative Example 3 by BCA assay.
FIG. 18 is an absorbance chart of the concentrated liquids prepared in Example 4 and Comparative Example 3.

### Description of Embodiments

Hereinafter, an example of the present disclosure will be explained with reference to the drawings. However, each of the configurations, combinations thereof, and the like in each embodiment are an example, and various additions, omissions, substitutions, and other changes may be made as appropriate without departing from the spirit of the present disclosure. The present disclosure is not limited by the embodiments and is limited only by the claims. In addition, in a case where a plurality of upper limit values and lower limit values are described for a specific parameter, any upper limit value and any lower limit value among these upper limit values and lower limit values can be combined to obtain a suitable numerical range.

### Purifying and Concentrating Apparatus of Liquid Containing Minute Useful Substance

The present disclosure relates to a purifying and concentrating apparatus of a liquid containing a minute useful substance. The purifying and concentrating apparatus according to the present disclosure has a first embodiment and a second embodiment. Hereinafter, the purifying and concentrating apparatus according to the first embodiment of the present disclosure will be described with reference to the drawings.

### First Embodiment

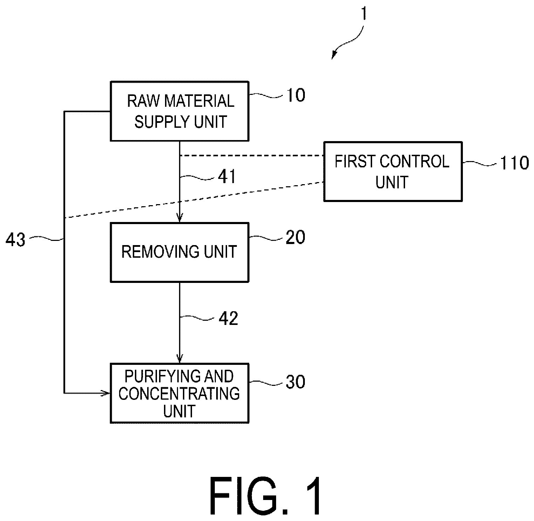

FIG. 1 is a schematic configuration diagram illustrating a purifying and concentrating apparatus 1 (hereinafter, referred to as the "purifying and concentrating apparatus 1") which is an example of the first embodiment of the present disclosure. The purifying and concentrating apparatus 1 of the present disclosure includes: a raw material supply unit 10 that supplies a raw material containing a minute useful substance; a removing unit 20 that removes an impurity from a liquid; a purifying and concentrating unit 30 that purifies and concentrates the liquid supplied from the raw material supply unit 10 or the removing unit 20 with a hollow fiber membrane; and a first control unit 110 that controls a timing of flow of the liquid from the raw material supply unit 10 to the purifying and concentrating unit 30 or flow of the liquid from the removing unit 20 to the purifying and concentrating unit 30. The liquid in the raw material supply unit 10 is supplied to the removing unit 20 through a flow path 41 or supplied to the purifying and concentrating unit 30 through a flow path 43. The liquid in the removing unit 20 is supplied to the purifying and concentrating unit 30 through a flow path 42. The purifying and concentrating apparatus 1 according to the present disclosure can produce a liquid containing a minute useful substance with high purity and high concentration (a purified and concentrated liquid of a minute useful substance), and can more efficiently prepare the purified and concentrated liquid. It is also useful as an apparatus capable of purifying and concentrating a minute useful substance by automatic control. Note that in the present disclosure, a "flow path" refers to a conduit through which a liquid flows.

### Raw material supply unit

The raw material supply unit 10 stores a raw material liquid containing a minute useful substance (hereinafter referred to as the "raw material liquid"). The raw material liquid in the raw material supply unit 10 is supplied to the removing unit 20 or the purifying and concentrating unit 30 depending on a purity thereof, on the opposite side of the coin, a concentration of an impurity in the raw material liquid. In a case where the concentration of an impurity in the raw material liquid is high, it can be set that the raw material liquid is supplied to the removing unit 20, and in a case where the concentration of an impurity in the raw material liquid is low, it can be set that the raw material liquid is supplied to the purifying and concentrating unit 30. The flow path 41 for supplying the raw material liquid to the removing unit 20 and the flow path 43 for supplying the raw material liquid to the purifying and concentrating unit 30 are connected to the raw material supply unit 10.

The raw material supply unit 10 includes a container that stores the raw material liquid. The container may be fixed to the purifying and concentrating apparatus 1, or may be installed to be easily detachable. In a case where the container is installed to be easily detachable, the raw material supply unit 10 can include various types of equipment such as a fixing member for fixing the container.

A shape and a volume of the container that stores the raw material liquid are not particularly limited, and can be determined depending on a type of the minute useful substance, an amount of the raw material liquid, and the like. In one embodiment, in a case of purifying and concentrating a raw material liquid containing a minute useful substance selected from an extracellular vesicle, an antibody, a virus, a protein, an enzyme, a nucleic acid, and the like, the volume of the container may be 50 to 5000 mL or 200 to 2000 mL.

### Removing unit

The removing unit 20 removes an impurity from the raw material liquid supplied from the raw material supply unit 10 through the flow path 41. According to this configuration, it is possible to efficiently prepare a purified and concentrated liquid of a minute useful substance. Hereinafter, the liquid from which the impurity has been removed in the removing unit 20 is referred to as a "roughly purified liquid".

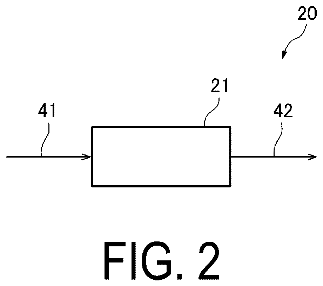

FIGS. 2 to 6 illustrate some typical schematic configuration examples of the removing unit 20.

The removing unit 20 illustrated in FIG. 2 is provided with a carrier column 21 that removes an impurity from the raw material liquid. One end of the carrier column 21 is connected to the flow path 41, and the other end is connected to the flow path 42 for supplying the roughly purified liquid to the purifying and concentrating unit 30. A flow path (described below) for discharging a liquid to a waste liquid tank (described below) may be connected to the flow path 42 from the carrier column 21.

Instead of the carrier column 21, the removing unit 20 may be provided with a device including a container filled with an adsorbent capable of adsorbing and removing an impurity from the raw material liquid. In the containers, an impurity in the raw material liquid can be adsorbed to the adsorbent by an action of stirring, vibration, shaking, or the like to remove or reduce the impurities. As such a device, a rotator can be used. The filler with the adsorbed impurity can be removed by an optional filtration unit.

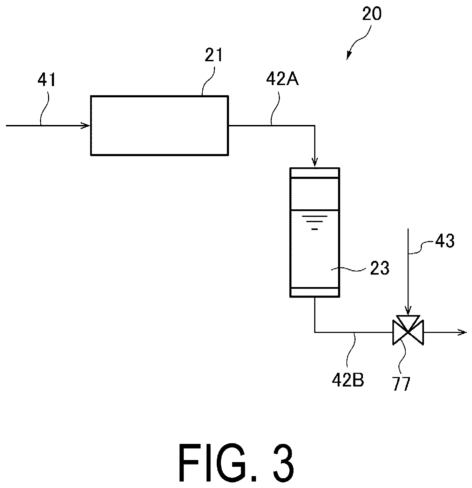

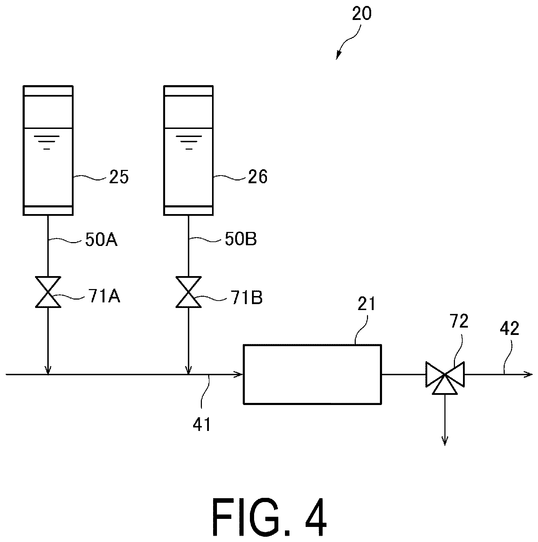

The removing unit 20 illustrated in FIG. 3 includes the carrier column 21 and a first tank 23 that stores the roughly purified liquid treated by the carrier column 21. The first tank 23 is disposed in the flow path 42. That is, the flow path 42 is disposed to be divided into a flow path 42A positioned on the carrier column 21 side and a flow path 42B positioned on the purifying and concentrating unit side across the first tank 23. The roughly purified liquid is stored in the first tank 23 from the carrier column 21 through the flow path 42A, and the roughly purified liquid stored in the first tank 23 is supplied to the purifying and concentrating unit 30 through the flow path 42B. A three-way valve 77 is provided in the flow path 42B. The first control unit 110 opens the three-way valve 77 in the flow path 42B toward the purifying and concentrating unit 30 depending on a treatment state of the roughly purified liquid in the purifying and concentrating unit 30, and supplies the roughly purified liquid in the first tank 23 to the purifying and concentrating unit 30. According to this configuration, the roughly purified liquid can be temporarily stored in the first tank 23 and then supplied to the purifying and concentrating unit 30. Thus, an impurity removal treatment in the removing unit 20 and a purifying and concentrating treatment in the purifying and concentrating unit 30 can be performed in parallel. As a result, the purifying and concentrating treatment can be efficiently performed. The configuration of the removing unit 20 illustrated in FIGS. 4 to 6 will be described below.

### Carrier column

The carrier column 21 is filled with a material capable of removing an impurity other than the minute useful substance in the raw material liquid. The material is preferably an adsorbent capable of adsorbing and removing an impurity from the raw material liquid.

The adsorbent is preferably a porous granular material capable of adsorbing and holding the impurity. The porous granular material refers to a granular material having a large number of pores on the surface and inside thereof, and a porous granular material of a polymer or a porous granular material of an inorganic material can be used. In a case where the minute useful substance contained in the raw material liquid to be treated by the purifying and concentrating apparatus 1 is a biological substance such as an extracellular vesicle, an antibody, a virus, a protein, an enzyme, or a nucleic acid, the porous granular material is preferably a material in which insides of pores are positively charged.

For these porous granular materials, two or more porous granular materials having one or different adsorption performances may be used in combination.

Examples of the two or more porous granular materials having different adsorption performances include porous granular materials having different pore sizes, porous granular materials having different specific surface areas, porous granular materials having different average particle sizes, porous granular materials having different types of substrates constituting the porous granular materials, and porous granular materials having different types and densities of functional groups present on inner surfaces of the pores.

Examples of the substrate of such a porous granular material include polysaccharides such as cellulose, agarose, starch, amylose, dextran, pullulan, and glucomannan; polyacrylic acids or derivatives thereof; synthetic polymers such as polyvinyl alcohol, nylon, polysulfone, polyacrylonitrile, polyethylene, polypropylene, and polystyrene; glass; porous glass; silica gel; and hydroxyapatite. One type of these may be used alone, or two or more types thereof may be used in combination. The porous granular material may also be a mixed-mode carrier with ion exchange and size exclusion, and preferably a mixed-mode carrier with cation exchange and size exclusion.

The porous granular material may be selected from those having an average pore size in a range from 0.1 to 1000 nm, depending on the type and the like of an impurity contained in the raw material liquid. Note that the average pore size can be measured by mercury intrusion porosimetry or inverse size-exclusion chromatography.

The porous granular material may be selected from those having an average particle size in a range from 1 to 1000 µm depending on the type and the like of an impurity contained in the raw material liquid. The average particle size can be measured by a laser diffraction/scattering method.

In one embodiment, in a case where the minute useful substance contained in the raw material liquid to be treated by the purifying and concentrating apparatus 1 is a biological substance such as an extracellular vesicle, an antibody, a virus, a protein, an enzyme, or a nucleic acid, the carrier column 21 may include an antibody column. The "antibody column" refers to a column filled with a carrier on which an antibody capable of binding to an antigen (impurity) is immobilized. In a case where the raw material liquid contains the above-described biological substance, an antibody column filled with a carrier on which an antibody capable of binding to (adsorbing) an impurity other than the biological substance is immobilized can be employed. Note that a plurality of types of impurities are often contained in the raw material liquid containing the above-described biomolecule as the minute useful substance, and thus a plurality of antibody columns corresponding to the types of impurities may be used.

Alternatively, instead of the carrier column 21, a module (column module) prepared by combining a plurality of carrier columns may be employed. In a case where a column module is used, the types of adsorbents contained in the respective carrier columns may be the same or different from each other. Inner diameters and lengths of the carrier columns may be the same or different from each other.

The removing unit 20 illustrated in FIG. 4 includes a sixth tank 25 (cleaning liquid storage tank) that stores a cleaning liquid for cleaning the carrier column 21 and a seventh tank 26 (equilibrating liquid storage tank) that stores an equilibrating liquid for equilibrating the carrier column 21. In the removing unit 20 illustrated in FIG. 4, the sixth tank 25 is configured to supply the cleaning liquid to the flow path 41 via a flow path 50A. The seventh tank 26 is configured to supply the equilibrating liquid to the flow path 41 via a flow path 50B. On-off valves 71A and 71B are disposed in the flow paths 50A and 50B. A three-way valve 72 is provided in the flow path 42 through which the roughly purified liquid is supplied from the carrier column 21 to the purifying and concentrating unit 30.

In one embodiment, it is preferable that the carrier column 21 which has reached adsorption saturation by treating one batch of the raw material liquid be cleaned and equilibrated, and then the next one batch of the raw material liquid be treated. The cleaning and equilibrating treatment of the carrier column 21 can be performed by opening the on-off valve 71A provided in the flow path 50A and the on-off valve 71B provided in the flow path 50B and supplying the cleaning liquid from the sixth tank 25 and the equilibrating liquid from the seventh tank 26 to the carrier column 21. It is preferable that the cleaning liquid and the equilibrating liquid after treating the carrier column 21 be discharged from the three-way valve 72 provided in the flow path 42 to a waste liquid tank (to be described below).

### Cleaning liquid and equilibrating liquid

Examples of the cleaning liquid for cleaning the carrier column 21, which can be employed, include a sodium chloride aqueous solution, hydrochloric acid, citric acid, a sodium hypochlorite aqueous solution, and a sodium hydroxide aqueous solution. From the viewpoint of safety, it is preferable to use a sodium chloride aqueous solution. When the cleaning liquid flows into the carrier column 21, an impurity adhering to the adsorbent in the carrier column 21 is removed. After removal with the cleaning liquid, the carrier column 21 is equilibrated. Examples of the equilibrating liquid that can be employed include physiological saline, a phosphate buffer solution (PBS), a tris-hydrochloric acid buffer solution, a sodium citrate buffer solution, a citrate phosphate buffer solution, an acetate buffer solution, and a borate buffer solution.

Note that the material of the sixth tank 25 and the seventh tank 26, which store the cleaning liquid and the equilibrating liquid respectively, is not particularly limited, and may have the same configuration as that of the second tank 31 and the third tank 32 described below. It is preferable that the sixth tank 25 and the seventh tank 26 have transparency from the viewpoint of easy visual confirmation of the amount of the stored liquid in the tank.

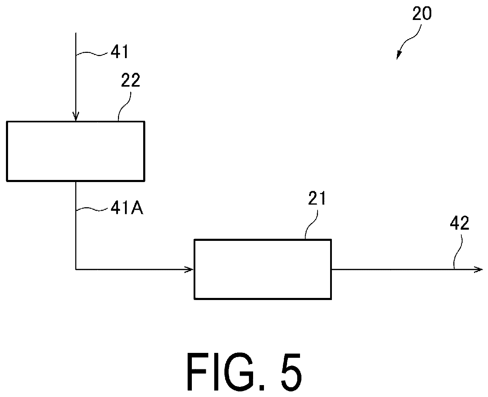

The removing unit 20 illustrated in FIG. 5 includes a filtration member that removes an impurity from the liquid before the liquid is supplied to the carrier column 21. The removing unit 20 illustrated in FIG. 5 has a configuration in which the filtration member 22 is interposed in the flow path 41. The raw material liquid filtered by the filtration member 22 is supplied to the carrier column 21 from the flow path 41A of the flow path 41 located on the carrier column 21 side across the filtration member 22. According to this configuration, the raw material liquid pretreated by the filtration member 22 can be supplied to the carrier column 21. Hereinafter, the raw material liquid treated by the filtration member 22 is referred to as a "pretreated liquid". Note that although the removing unit 20 in FIG. 5 is configured to include the filtration member 22 and the carrier column 21, it may be configured to include only the filtration member 22.

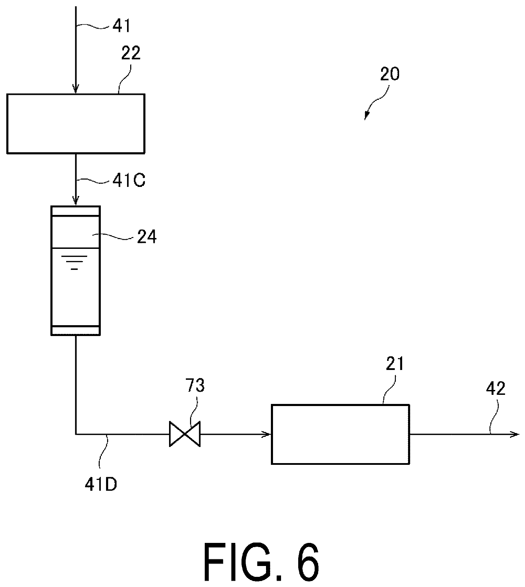

The removing unit 20 illustrated in FIG. 6 includes a tank 24 (pretreated liquid storage tank) that stores the pretreated liquid from the filtration member 22. The pretreated liquid from the filtration member 22 is stored in the tank 24 via a flow path 41C. The pretreated liquid stored in the tank 24 is supplied to the carrier column 21 via a flow path 41D. The first control unit 110 (not illustrated) can supply the pretreated liquid in the tank 24 to the carrier column 21 by opening an on-off valve 73 in the flow path 41D in accordance with the progress of the impurity removal treatment in the carrier column 21. According to this configuration, the pretreated liquid can be supplied to the carrier column 21 after being temporarily stored in the tank 24. Thus, the filtration treatment in the filtration member 22 and the impurity removal treatment in the carrier column 21 can be performed in parallel to efficiently perform the purifying and concentrating treatment, which is preferable.

In FIG. 6, the filtration member 22 is disposed below the raw material supply unit 10. According to this configuration, the supply of the raw material liquid from the raw material supply unit 10 to the filtration member 22 proceeds by the own weight of the raw material liquid, and thus a liquid feeding member (a pump or the like) for supplying the raw material liquid from the raw material supply unit 10 to the filtration member 22 can be omitted. Accordingly, a more compact purifying and concentrating apparatus can be provided, which is preferable.

### Filtration member

The filtration member 22 is a member for filtering the raw material liquid. In a case where the minute useful substance contained in the raw material liquid contains an extracellular vesicle, the filtration member 22 is preferably a filtration filter including a microfiltration membrane (microfiltration membrane module). From the viewpoint of efficiently filtering and removing an impurity such as a biomolecule larger than the extracellular vesicle of interest, such as a cell in the raw material liquid, the average pore size of the microfiltration membrane (microfiltration membrane module) is preferably from 0.10 to 0.50 µm, and more preferably from 0.15 to 0.30 µm. As the material of the microfiltration membrane, polyether sulfone, polyvinylidene fluoride, or the like is preferable. As the microfiltration membrane, a commercially available product including these materials can be used.

### First control unit

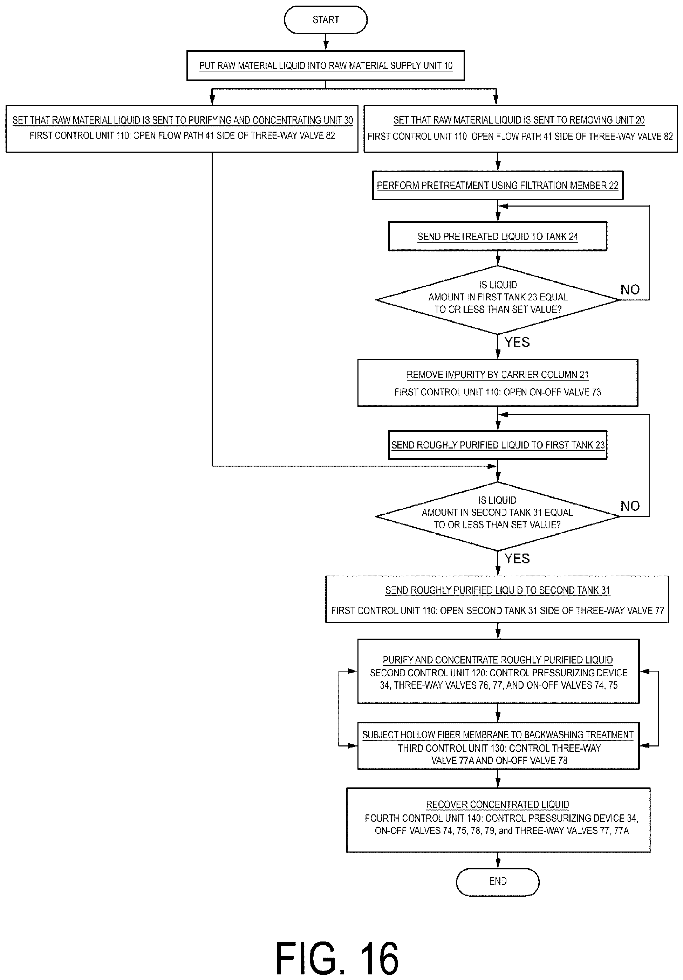

Next, the first control unit will be described in detail with reference to the flowchart of FIG. 16.

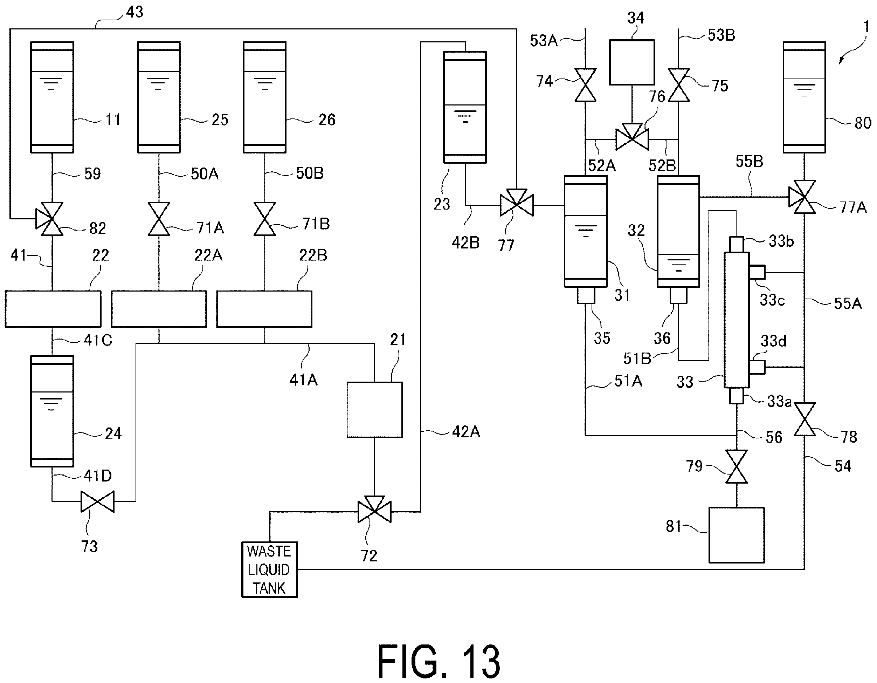

FIG. 16 is a basic flowchart in a case of performing the purifying and concentrating treatment using the purifying and concentrating apparatus 1 illustrated in FIG. 13. An example of liquid flow control in each control unit is illustrated in the flowchart of FIG. 16.

The first control unit 110 can supply the raw material liquid to the removing unit 20 or the purifying and concentrating unit 30 based on a result set by an impurity concentration of the raw material liquid in the raw material supply unit 10. In a case where the impurity concentration of the raw material liquid is high, settings are arranged so that the raw material liquid in the raw material supply unit 10 is supplied to the removing unit 20. In this case, the first control unit 110 opens a three-way valve 82 disposed below the container 11 in FIG. 13 to the flow path 41 side to supply the raw material liquid to the removing unit 20 (the filtration member 22 in FIG. 13). In a case where the impurity concentration of the raw material liquid is low, settings are arranged so that the raw material liquid in the raw material supply unit 10 is supplied to the purifying and concentrating unit 30. In this case, the first control unit 110 opens the three-way valve 82 to the flow path 43 side, and thus the raw material liquid can be fed toward the purifying and concentrating unit 30. The liquid treated by the filtration member 22 (pretreated liquid) is supplied to the tank 24. The first control unit 110 can supply the liquid from the tank 24 toward the carrier column 21 until the amount of the roughly purified liquid in the first tank 23 (roughly purified liquid storage tank) reaches a set value. Specifically, the pretreated liquid can be supplied to the carrier column 21 by opening the on-off valve 73 provided below the tank 24.

When the amount of the roughly purified liquid in the first tank 23 reaches a preset value, the roughly purified liquid is fed to the second tank 31. At this time, the first control unit 110 can also check liquid amounts in the second tank 31 and the third tank 32. In a case where the first control unit 110 determines that the liquid amounts in the second tank 31 and the third tank 32 are preset liquid amounts on the basis of liquid level monitoring by the in-tank liquid amount monitoring devices 35 and 36 and/or the camera, which will be described below, the three-way valve 77 connected to the flow path 42B is opened toward the second tank 31 side, whereby the roughly purified liquid in the first tank 23 can be supplied to the second tank 31.

The flow path 42B connected to the first tank 23 in FIG. 13 is provided with a level sensor (not illustrated) that determines a liquid amount in the tank. The first control unit 110 can determine whether or not the roughly purified liquid remains in the first tank 23 on the basis of the information of the level sensor. When it is determined that all the roughly purified liquid in the first tank 23 has been supplied to the second tank 31, the first control unit 110 can open the on-off valve 73 to supply the pretreated liquid in the tank 24 to the carrier column 21 again. When the flow of the liquid is controlled in this manner, it is possible to perform the cleaning and equilibrating treatment of the carrier column 21 each time the treatment of one batch is completed.

### Purifying and concentrating unit

The purifying and concentrating unit 30 purifies and concentrates the liquid supplied from the raw material supply unit 10 or the removing unit 20 with a hollow fiber membrane. The hollow fiber membrane is used to perform purifying and concentrating, and thus it is possible to prepare the liquid containing the minute useful substance with high purity and high concentration.

### Hollow fiber membrane

The material of the hollow fiber membrane of the present disclosure can be appropriately designed depending on the purpose of use of the present disclosure, the properties of the liquid containing the minute useful substance, and the like.

Specifically, an organic polymer-based material such as polyvinylidene fluoride (PVDF), polyether sulfone (PES), polysulfone (PSf), polyolefin (PE, PP, PTFE, etc.), polyamide, polyacrylonitrile, or cellulose acetate (CA), or a modified product thereof can be used.

In addition, a composite hollow fiber membrane including a plurality of components selected from the above can also be used.

Among them, in a case where the minute useful substance is a biomolecule such as an exosome, it is preferable to use a hollow fiber membrane made of cellulose acetate (CA) (that is, the hollow fiber membrane is preferably made of a resin material containing cellulose acetate). When the hollow fiber membrane made of cellulose acetate is used, it is possible to suppress a decrease in filtration rate during the purifying and concentrating treatment and to further improve the effect of recovering the minute useful substance deposited on a membrane surface of the hollow fiber membrane. The reason for this is as follows. The surface charge of the biomolecule is a weak negative charge and the cellulose acetate membrane also has a weak negative charge. Thus, the biomolecule and the cellulose acetate membrane are not attracted to each other by a strong Coulomb force, and the shear force of the cross-flow filtration easily functions by appropriately controlling a circulation linear velocity. In addition, when the cellulose acetate membrane is used, it is possible to more efficiently recover the minute useful substance deposited on the membrane surface of the hollow fiber membrane by a method described below.

The material of the hollow fiber membrane of the present disclosure can be appropriately designed depending on the purpose of use of the present disclosure, the properties of the raw material liquid containing the minute useful substance, and the like. The hollow fiber membrane can also be coated with an appropriate substance to improve fouling resistance of the surface of the hollow fiber membrane, depending on the properties of the raw material liquid containing the minute useful substance.

The coating treatment can be performed, for example, by the same method as the method of bonding a cationic polymer to the surface of a hollow fiber membrane made of sulfonated polyether sulfone and polyether sulfone described in Japanese Patent No. 6081290.

That is, there can be used a method in which the hollow fiber membrane is immersed in an aqueous solution (aqueous dispersion) of a coating substance, or a method in which an aqueous solution (aqueous dispersion) of a coating substance is caused to pass through the hollow fiber membrane in a state of the hollow fiber membrane or hollow fiber membrane module to adsorb and bond the coating substance to the surface of the hollow fiber membrane, and then the coating substance just deposited on the thin layer of the coating substance is removed by washing with water.

In a case of concentrating and purifying a certain type of extracellular vesicle in a certain type of culture supernatant, it may be preferable to coat a hollow fiber membrane made of cellulose acetate with a certain type of biomolecule, in terms of purifying and concentrating efficiency and improvement in recovery rate of the minute useful substance such as an extracellular vesicle.

As an example, a cellulose acetate hollow fiber membrane module (molecular weight cutoff of 300000, membrane area of 0.062 m²) is subjected to coating treatment in which 1.3 mg/mL of fat-free milk powder solution prepared by dissolving fat-free milk powder in phosphate buffer solution (PBS) is caused to pass therethrough at a liquid flow rate of 200 mL/min for 10 minutes to adsorb and bind biomolecules such as casein in the fat-free milk powder to the surface of the hollow fiber membrane as a thin layer. Then, the hollow fiber membrane module is washed with 250 mL of phosphate buffer solution (PBS) to remove excess components just deposited on the thin layer. The resulting hollow fiber membrane module can be used.

In one embodiment, from the viewpoint of enhancing the fouling resistance of the hollow fiber membrane, a biomolecule of a type different from the minute useful substance is preferably attached to at least a partial region of the hollow fiber membrane. The biomolecule is more preferably attached to a half or more region of the surface of the hollow fiber membrane, and is preferably attached to the entire region. In one embodiment, a layer containing a biomolecule of a type different from the minute useful substance is preferably formed on at least a part of the surface of the hollow fiber membrane. The "layer containing a biomolecule" means that a substance containing a biomolecule is attached to at least a partial region of the hollow fiber membrane with a certain breadth. The layer containing a biomolecule is preferably formed in a half or more region of the surface of the hollow fiber membrane, and more preferably formed in the entire region. In one embodiment, at least a part of the surface of the hollow fiber membrane is preferably coated with a biomolecule of a type different from the minute useful substance.

The biomolecule of a type different from the minute useful substance is prepared from an animal cell tissue such as blood, milk, or cartilage, or another biological cell tissue. Typical examples thereof include biomolecules contained in skim milk, fat-free milk powder, and the like (e.g., casein); biomolecules contained in serum and the like (e.g., serum globulin, serum albumin); and gelatin (e.g., fish gelatin), and may be one or more selected from these.

### Average inner diameter of hollow fiber membrane

The average inner diameter of the hollow fiber membrane of the present disclosure can be appropriately designed depending on the purpose of use of the present disclosure, the properties of the liquid containing the minute useful substance, and the like. In one embodiment, a hollow fiber membrane having an average inner diameter of from 0.2 mm to 1.4 mm can be employed. In another embodiment, a hollow fiber membrane having an average inner diameter of from 0.2 mm to 2.0 mm can be employed. Note that the "average inner diameter of the hollow fiber membrane" in the present disclosure is a value determined by cutting one hollow fiber membrane along any plane perpendicular to the longitudinal direction and calculating from the diameter of the smallest circle inscribed in the hollow portion of the cut surface. In the present disclosure, the diameter is measured for any 10 or more and 100 or less cut surfaces, and an average value of the measured values is defined as the "average inner diameter".

Note that the hollow fiber membrane of the present disclosure may have an average outer diameter of from 0.3 mm to 2.0 mm. In one embodiment, in a case where the average inner diameter of the hollow fiber membrane is from 0.2 mm to 1.0 mm, the average outer diameter may be from 0.3 mm to 1.4 mm. In another embodiment, in a case where the average inner diameter of the hollow fiber membrane is from 1.0 mm to 1.4 mm, the average outer diameter may be from 1.5 mm to 2.0 mm. Note that the "average outer diameter of the hollow fiber membrane" is a value calculated from the diameter of the smallest circle surrounding the outer edge of the cut surface of the hollow fiber membrane cut by the same method as the above-described average inner diameter of the hollow fiber membrane. In the present disclosure, the diameter is measured for any 10 or more and 100 or less cut surfaces, and an average value of the measured values is defined as the "average outer diameter".

In the purpose of use of the present disclosure, the average inner diameter of the hollow fiber membrane may be from 0.6 mm to 1.4 mm from the viewpoint of easily ensuring a thickness at which the hollow fiber membrane does not become excessively flexible and easily improving the recovery efficiency of the minute useful substance deposited on the membrane surface of the hollow fiber membrane.

Further, the average inner diameter may be adjusted by a liquid viscosity of the liquid containing the minute useful substance during operation. In a case where the liquid viscosity is high (for example, in a case where the liquid viscosity at a liquid temperature of 20°C and a shear rate of 25 s⁻¹ is from 10 to 30 mPa·s), it is preferable to use a large-diameter hollow fiber membrane having an average inner diameter of 1.0 mm to 1.4 mm. In a case where the liquid viscosity of the liquid containing the minute useful substance during operation is low (for example, in a case where the liquid viscosity at a liquid temperature of 20°C and a shear rate of 25 s⁻¹ is less than 10 mPa·s), it is preferable to use hollow fiber membranes having an average inner diameter of 0.6 mm to 1.0 mm. Note that when the average inner diameter of the hollow fiber membranes increases, an effective membrane area tends to decrease in a case of using the hollow fiber membranes as a hollow fiber membrane module. Accordingly, it is preferable to use hollow fiber membranes having an average inner diameter of from 0.2 mm to 1.0 mm from the viewpoint of easily suppressing a decrease in effective membrane area in a case of being formed into the hollow fiber membrane module.

### Molecular weight cutoff of hollow fiber membrane

The molecular weight cutoff of the hollow fiber membrane of the present disclosure can be appropriately designed depending on the purpose of use of the present disclosure, the properties of the raw material liquid containing the minute useful substance, and the like. In one embodiment, the molecular weight cutoff may be from 5000 to 3000000, or from 10000 to 1000000. In a case where the minute useful substance is a specific protein, it is preferable to use a hollow fiber membrane having a molecular weight cutoff slightly lower than the molecular weight of the protein (for example, a hollow fiber membrane having a molecular weight cutoff about 10 to 50% lower than the molecular weight of the protein). In one embodiment, in a case where the minute useful substance is an exosome, the molecular weight cutoff of the hollow fiber membrane is preferably from 50000 to 1000000, more preferably from 70000 to 800000, and even more preferably from 100000 to 500000.

In a case where the minute useful substance is an extracellular vesicle, a γ-globulin permeability of the hollow fiber membrane module of the present disclosure is preferably from 5 to 90%, and more preferably from 10 to 80%, from the viewpoint of efficiency of purifying and concentrating. In the present disclosure, a method for measuring a permeability of an impurity in the liquid containing the minute useful substance, including the above-described γ-globulin, is not particularly limited, and a method known in the related art can be employed. Specifically, it is possible to employ a method of measuring a permeability of a standard substance having a molecular structure similar to that of the impurity and a molecular weight similar to that of the impurity. In a case of measuring the permeability, the hollow fiber membrane module may be used, or one hollow fiber membrane used in the hollow fiber membrane module may be directly used. Furthermore, the permeability of an internal pressure type hollow fiber membrane module can be substituted by a permeability measured by a method in which in one hollow fiber membrane, pressure is applied from the outside of the hollow fiber membrane to allow permeation to the inside.

### Hollow fiber membrane module

The purifying and concentrating unit 30 in the present disclosure may have a configuration in which a hollow fiber membrane module including a plurality of hollow fiber membranes accommodated in a container having a liquid inlet/outlet is provided. The container has at least a first liquid inlet/outlet 33a and a second liquid inlet/outlet 33b for supplying a liquid to the hollow fiber membrane, and third liquid inlets/outlets 33c and 33d for discharging a liquid having permeated through the hollow fiber membrane.

The number of hollow fiber membranes used in the hollow fiber membrane module of the present disclosure is not particularly limited. Depending on the purpose of use of the present disclosure, a weight of the minute useful substance to be purified and concentrated, and the like, the number of hollow fiber membranes to be incorporated into the module can be appropriately designed to provide a necessary effective membrane area.

The hollow fiber membranes are preferably bundled with an adhesive or the like and accommodated in the container. In the present disclosure, when the liquid containing the minute useful substance is supplied from the first liquid inlet/outlet 33a and/or the second liquid inlet/outlet 33b of the hollow fiber membrane module, the liquid flows to one side of the hollow fiber membrane bundle (inside or outside of the hollow fiber membrane bundle) in the container, and is purified and concentrated by the hollow fiber membrane bundle. The liquid that has permeated through the hollow fiber membrane bundle flows out to the other side of the hollow fiber membrane bundle (outside or inside of the hollow fiber membrane bundle) and is discharged from the third liquid inlets/outlets 33c and 33d. At this time, the liquid remaining in the inside or outside of the hollow fiber membrane bundle without permeating can be discharged (recovered) from the first liquid inlet/outlet and/or the second liquid inlet/outlet to the outside of the hollow fiber membrane module. Hereinafter, in the present disclosure, a liquid that has permeated through the hollow fiber membrane is referred to as a "permeated liquid", and a liquid that has not permeated through the hollow fiber membrane and remains in the hollow fiber membrane is referred to as a "concentrated liquid". Note that the "concentrated liquid" refers to any liquid purified and concentrated by a hollow fiber membrane.

A hollow fiber membrane module in which the liquid containing the minute useful substance is supplied to the inside of the hollow fiber membrane bundle is an internal pressure type hollow fiber membrane module, and a hollow fiber membrane module in which the liquid containing the minute useful substance is supplied to the outside of the hollow fiber membrane bundle is an external pressure type hollow fiber membrane module. Hereinafter, an example of the internal pressure type hollow fiber membrane module will be described as an embodiment of the hollow fiber membrane module in the present disclosure.

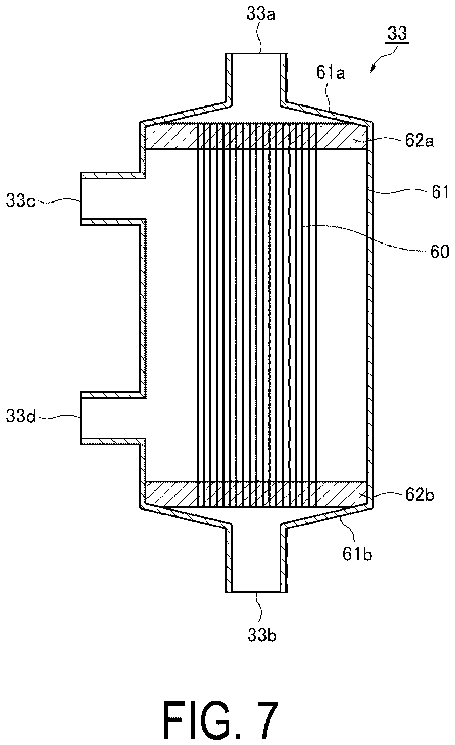

FIG. 7 is a schematic cross-sectional view illustrating an example of an internal pressure type hollow fiber membrane module 33. In the hollow fiber membrane module 33, a hollow fiber membrane bundle 60 in which a plurality of hollow fiber membranes are bundled is accommodated in a cylindrical container 61. The plurality of hollow fiber membranes are bundled with a biocompatible sealing adhesive (for example, a polyurethane-based sealing adhesive or the like) to form the hollow fiber membrane bundle 60, and are bonded and fixed to both ends of the inside of the cylindrical container 61 in a longitudinal direction thereof with adhesive layers 62a and 62b made of the sealing adhesive. From the viewpoint of transparency, the cylindrical container 61 may be made of a material such as polycarbonate, poly(meth)acrylate, or polysulfone. Note that the plurality of hollow fiber membranes are open at both end surfaces 61a and 61b of the cylindrical container 61. The first liquid inlet/outlet 33a and the second liquid inlet/outlet 33b are provided at both ends of the hollow fiber membrane module 33 in the longitudinal direction. In addition, the third liquid inlets/outlets 33c and 33d are provided in a side surface portion of the hollow fiber membrane module 33 in the longitudinal direction. The liquid supplied from the first liquid inlet/outlet 33a and/or the second liquid inlet/outlet 33b is fed to the inside of the hollow fiber membrane bundle 60 to be purified and concentrated. The permeated liquid that has permeated to the outside of the hollow fiber membrane bundle 60 is discharged from the third liquid inlets/outlets 33c and 33d provided on the side surface of the cylindrical container 61. Note that the third liquid inlets/outlets are preferably provided at least two positions, that is, an upper portion and a lower portion of the side surface of the cylindrical container 61. In FIG. 7, the third liquid inlets/outlets 33c and 33d are provided only on one side surface portion of the hollow fiber membrane module 33, but the present disclosure is not limited thereto. The third liquid inlets/outlets 33c and 33d may be provided separately on one side surface portion and the other side surface portion.

Although FIG. 7 illustrates the internal pressure type hollow fiber membrane module, the hollow fiber membrane module of the present disclosure may be of an external pressure type as described above. In a case of the external pressure type, it is necessary to appropriately design installation positions and installation methods of the first to third liquid inlets/outlets.

### Filling rate in hollow fiber membrane module

A filling rate in the hollow fiber membrane module of the present disclosure is preferably from 5 to 55%, more preferably from 20 to 55%, and still more preferably from 25 to 55%. The filling rate refers to a percentage of the total sum of cross-sectional areas of outer diameter portions of the hollow fiber membrane bundle in the cross-sectional area of the hollow fiber membrane module, generally in the transverse direction of a cylindrical container. When the filling rate is from 5 to 55%, the minute useful substance deposited on the membrane surfaces of the hollow fiber membranes can be more efficiently recovered while optimizing the size of the hollow fiber membrane module.

Hereinafter, an aspect in which the purifying and concentrating unit 30 of the present disclosure includes the hollow fiber membrane module 33 will be described with reference to the drawings.

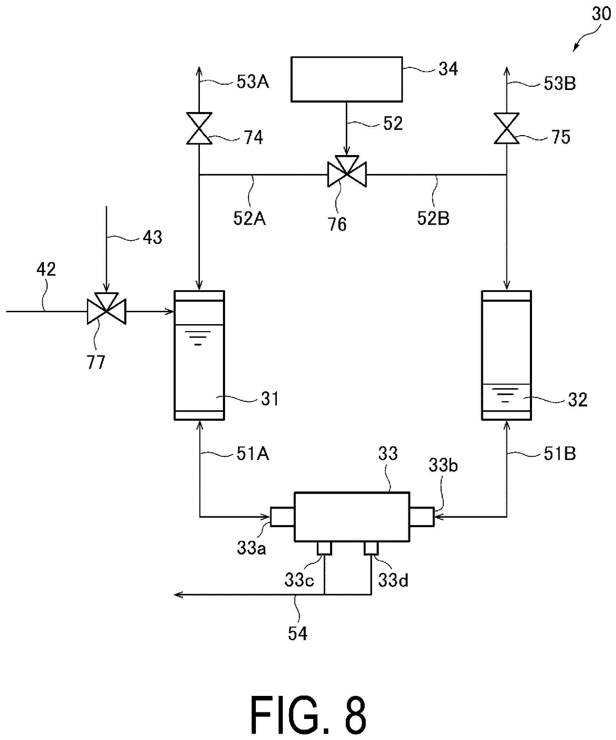

FIGS. 8 to 11 illustrate some typical schematic configuration diagrams of the purifying and concentrating unit 30. Note that the purifying and concentrating apparatus 1 of the present disclosure may include a valve, an automatic valve, a backflow prevention valve, an orifice, a flowmeter, a pressure gauge, a liquid level gauge, a safety device such as a rupture disk, and the like, which are omitted in the schematic configuration diagram.

The purifying and concentrating unit 30 illustrated in FIG. 8 includes the second tank 31 and the third tank 32, the hollow fiber membrane module 33 connected to the second tank 31 and the third tank 32, and a pressurizing device 34 that pressurizes the second tank 31 and the third tank 32. The pressurizing device 34 is connected to a three-way valve 76 via a gas supply path 52.

The second tank 31 is a tank that stores the liquid supplied from the raw material supply unit 10 or the removing unit 20, that is, the raw material liquid or the roughly purified liquid, and is connected to the first liquid inlet/outlet 33a of the hollow fiber membrane module 33 via a flow path 51A. Similarly to the second tank 31, the third tank 32 is a tank that stores the raw material liquid or the roughly purified liquid, and is connected to the second liquid inlet/outlet 33b of the hollow fiber membrane module 33 via a flow path 51B. Upper portions of the second tank 31 and the third tank 32 are connected to the pressurizing device 34. A second control unit 120 (not illustrated) performs control in such a manner that the pressurizing device 34 is operated and the three-way valve 76 is switched to selectively pressurize either the second tank 31 or the third tank 32, whereby the raw material liquid or the roughly purified liquid in the second tank 31 or the third tank 32 is alternately (selectively) supplied to the hollow fiber membrane module 33. The liquid discharged from the second tank 31 or the third tank 32 is purified and concentrated by the hollow fiber membrane module 33, and then fed to another tank. Hereinafter, the liquid stored in the second tank 31 before completion of the purifying and concentrating treatment is referred to as a "second stored liquid", and the liquid stored in the third tank 32 before completion of the purifying and concentrating treatment is referred to as a "third stored liquid". Note that although the flow paths 42 and 43 are connected to the second tank 31 in FIG. 8, the flow paths 42 and 43 may be connected to the third tank 32. Note that the second control unit 120 will be described in detail below.

### Pressurizing device

The pressurizing device 34 of the present disclosure supplies gas from a gas supply source into the second tank 31 and the third tank 32. The gas for pressurization is supplied to the second tank 31 or the third tank 32 from gas supply paths 52A and 52B provided with pressure gauges (not illustrated) by switching the three-way valve 76 by the second control unit 120 (not illustrated). The gas can be selected from inert gases such as nitrogen, argon, and helium, carbon dioxide, filtered air, and the like.

In a case of pressurizing the second tank 31 by the pressurizing device 34, the second control unit 120 (not illustrated) supplies the gas from the gas supply source (not illustrated) to the gas supply path 52, and switches the three-way valve 76 to send the gas to the gas supply path 52A. At this time, an on-off valve 74 of a gas vent path 53A and the three-way valve 77 connecting the flow paths 42 and 43 are closed, and an on-off valve 75 of a gas vent path 53B is opened. On the other hand, in a case of pressurizing the third tank 32 by the pressurizing device 34, the second control unit 120 supplies the gas from the gas supply source (not illustrated) to the gas supply path 52 and switches the three-way valve 76 to send the gas to the gas supply path 52B. At this time, the on-off valve 75 in the gas vent path 53B and the three-way valve 77 are closed, and the on-off valve 74 of the gas vent path 53A is opened. These operations allow the pressurizing device 34 to selectively pressurize the second tank 31 and the third tank 32.

### Second tank and third tank

In the purifying and concentrating apparatus 1 of the present disclosure, the second tank 31 and the third tank 32 preferably have transparency, whereby liquid level positions of the second stored liquid and the third stored liquid can be observed visually or with a camera described below. In addition, from the viewpoint of preventing the second stored liquid or the third stored liquid from adhering to and remaining on the inner wall surface of the tank, the second tank 31 and the third tank 32 are preferably formed of a material having water repellency. Specifically, the second tank 31 and the third tank 32 are preferably made of a material containing polyacrylonitrile; poly(meth)acrylate such as poly(meth)acrylic acid ester; polycarbonate; a fluorine resin; or the like. Note that in a case where a liquid level of a transparent portion of the liquid inlet/outlet of the second tank 31 or the third tank 32 is managed by an in-tank liquid amount monitoring device or the like described below, the second tank 31 and the third tank 32 may be made of an opaque material such as a metal or a fiber-reinforced resin.

In the purifying and concentrating unit 30 illustrated in FIG. 8, the second stored liquid enters the hollow fiber membrane module 33 from the first liquid inlet/outlet 33a via the flow path 51A, is purified and concentrated by the hollow fiber membrane bundle 60, is then discharged from the second liquid inlet/outlet 33b, and is fed to the third tank 32. In this case, the second stored liquid is purified and concentrated by tangential flow filtration in which the liquid flows in parallel with the membrane surface of the hollow fiber membrane bundle 60 in the longitudinal direction. The permeated liquid having permeated through the hollow fiber membrane bundle 60 is discharged to a waste liquid tank (described below) from a flow path 54 connected to the third liquid inlets/outlets 33c and 33d of the hollow fiber membrane module 33.

Similarly, the third stored liquid enters the hollow fiber membrane module 33 from the second liquid inlet/outlet 33b via the flow path 51B, is purified and concentrated by the hollow fiber membrane bundle 60, and is then discharged from the first liquid inlet/outlet 33a to be fed to the second tank 31. Also in this case, the third stored liquid is purified and concentrated by tangential flow filtration in which the liquid flows in parallel with the membrane surface of the hollow fiber membrane bundle 60 in the longitudinal direction. When these operations are alternately repeated, the minute useful substance and the impurity are hardly deposited on the surface of the hollow fiber membrane. As a result, it is possible to suppress a decrease in liquid permeation rate and to maintain high treatment efficiency. That is, when the liquid is subjected to alternating tangential flow filtration, it is possible to more efficiently produce the liquid containing the minute useful substance with higher purity and higher concentration.

The second control unit 120 sets amounts of the second stored liquid and the third stored liquid, and controls the pressurizing device 34 and the on-off valve in such a manner that the second stored liquid and the third stored liquid have the set values, whereby the second tank 31 or the third tank 32 can be alternately pressurized.

The purifying and concentrating unit 30 in FIG. 8 may be configured to include a device for monitoring liquid amounts in the second tank 31 and the third tank 32. According to this configuration, the liquid amounts in the second tank 31 and the third tank 32 can be grasped, and thus the alternating tangential flow filtration can be performed more efficiently.

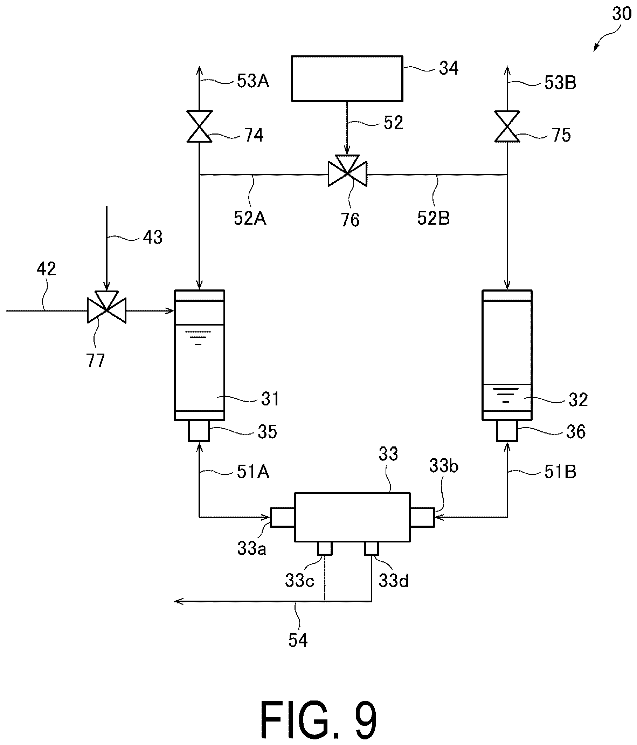

The purifying and concentrating unit 30 illustrated in FIG. 9 has the same configuration as that of the purifying and concentrating unit 30 illustrated in FIG. 8, and further, the second tank 31 and the third tank 32 are respectively provided with in-tank liquid amount monitoring devices 35 and 36 each including an optical sensor (not illustrated) at a liquid inlet/outlet of the tank bottom portion. Each of the in-tank liquid amount monitoring devices 35 and 36 is configured to, by the optical sensor, irradiate the liquid inlet/outlet of the corresponding one of the second tank 31 and the third tank 32 with light, detect the transmittance of the light at the liquid inlet/outlet, and monitor the liquid amount in the tank based on a change in the transmittance. The second control unit 120 (not illustrated) determines the liquid amounts of the second stored liquid and the third stored liquid in the second tank 31 and the third tank 32 by the in-tank liquid amount monitoring devices 35 and 36, and determines the tank to be pressurized by the pressurizing device 34.

### In-tank liquid amount monitoring device

The in-tank liquid amount monitoring devices 35 and 36 of the present disclosure each include an optical sensor. The optical sensor includes a light-emitting element and a light-receiving element. Light is emitted from the light-emitting element toward the liquid inlet/outlet at the tank bottom portion, and the light-receiving element detects intensity (transmittance) of the transmitted light. Note that a portion of the liquid inlet/outlet at the tank bottom portion, which is irradiated with light, is made of a transparent material. The detected transmittance is further sent to the detection unit. When the detection unit detects a change in transmittance, the second control unit 120 changes the tank to be pressurized by the pressurizing device 34.

The relationship between the in-tank liquid amount monitoring devices and the second control unit will be described more specifically.

In a case where the second tank 31 is pressurized by the pressurizing device 34 and the second stored liquid is supplied toward the third tank 32, when the amount of the second stored liquid in the second tank 31 decreases, gas supplied from the pressurizing device 34 is discharged from the liquid inlet/outlet of the second tank 31, and the transmittance of the light in the liquid inlet/outlet changes. When the in-tank liquid amount monitoring device 35 detects the change in transmittance, the second control unit 120 (not illustrated) switches the three-way valve 76 to change the tank to be pressurized by the pressurizing device 34 from the second tank 31 to the third tank 32. Similarly, when the in-tank liquid amount monitoring device 36 provided at the liquid inlet/outlet of the third tank 32 detects a change in transmittance, the second control unit 120 switches the three-way valve 76 to change the tank to be pressurized by the pressurizing device 34 from the third tank 32 to the second tank 31. The in-tank liquid amount monitoring devices 35 and 36 may be fiber sensors. In this way, the second control unit 120 can perform control in such a manner that the liquids in the second tank 31 and the third tank 32 are alternately supplied to the hollow fiber membrane module.

In other embodiments, the liquid amount in the tank may be monitored on the basis of an image captured by a camera disposed in the purifying and concentrating unit 30. That is, the purifying and concentrating unit 30 may include a camera that monitors the liquid level positions in the second tank 31 and the third tank 32, and the second control unit 120 may grasp the liquid amounts in the tanks from information on the liquid level positions in the second tank 31 and the third tank 32 output from the camera and determine the tank to be pressurized by the pressurizing device 34. Specifically, the purifying and concentrating unit 30 is provided with a camera at a position where the liquid levels in the second tank 31 and the third tank 32 can be observed, and an image of the liquid level positions output from the camera is transmitted to the detection unit. The detection unit determines the liquid amounts in the second tank 31 and the second tank 31 from the information on the liquid level positions. For example, when the detection unit determines that an amount of the second stored liquid in the second tank 31 is equal to or less than a preset amount from the information on the liquid level positions by the camera, the second control unit 120 switches the three-way valve 76 to change the tank to be pressurized by the pressurizing device 34 from the second tank 31 to the third tank 32. Similarly, when the detection unit determines that an amount of the third stored liquid in the third tank 32 is equal to or less than a preset amount, the second control unit 120 switches the three-way valve 76 to change the tank to be pressurized by the pressurizing device 34 from the third tank 32 to the second tank 31.

### Second control unit

An example of determination in liquid flow control of the second control unit 120 is illustrated in the flowchart of FIG. 16. As illustrated in FIG. 16, the second control unit 120 can operate the on-off valve and the pressurizing device mainly to cause the liquid (the roughly purified liquid or the raw material liquid) to flow between the second tank 31 and the third tank 32. In addition, the second control unit 120 can determine the liquid amounts in the second tank 31 and the third tank 32 on the basis of the information from the in-tank liquid amount monitoring devices 35 and 36 and/or the camera, and cause the liquid in the tanks to flow. The second control unit 120 can also set the liquid amount in the second tank 31 and/or the third tank 32. An example of a method for setting the liquid amount in the tank (setting of a management liquid level position C) will be described below.

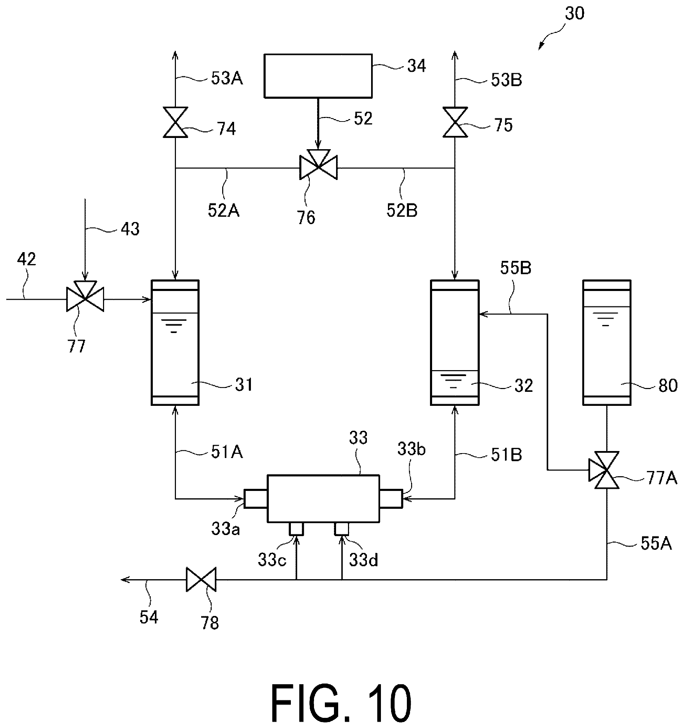

The purifying and concentrating unit 30 in FIG. 10 includes a cleaning tank that supplies a diluent to the hollow fiber membrane module 33 to clean a plurality of hollow fiber membranes in the hollow fiber membrane bundle 60. The purifying and concentrating unit 30 illustrated in FIG. 10 includes a fourth tank 80 that stores a diluent, the fourth tank being connected to a flow path 55A through which the diluent is supplied to the third liquid inlets/outlets 33c and 33d of the hollow fiber membrane module 33; and a third control unit 130 (not illustrated) configured to control a timing of supplying the diluent in the fourth tank 80 to the third liquid inlets/outlets 33c and 33d of the hollow fiber membrane module 33. A flow path 55B through which the diluent in the tank is directly supplied to the third tank 32 (or the second tank 31) is connected to the fourth tank 80.

### Third control unit

The third control unit 130 controls flow of the diluent in the fourth tank 80. An example of determination in liquid flow control of the third control unit 130 is illustrated in the flowchart of FIG. 16. The third control unit 130 can supply the diluent from the fourth tank 80 to either the third tank 32 or the hollow fiber membrane module 33 during the purifying and concentrating treatment of the liquid. Hereinafter, supplying the diluent to the third tank 32 will be referred to as a "dilution treatment", and supplying the diluent to the hollow fiber membrane module 33 will be referred to as a "backwashing treatment".

### Diluent

As the diluent, for example, pure water; filtered water of tap water, ground water, seawater, river water, or the like; various aqueous solutions; or the like can be used depending on the purpose of the present disclosure and the type of the minute useful substance. In a case where the minute useful substance is an exosome, physiological saline or a medical or biochemical buffer solution is preferable. As the buffer solution, for example, a phosphate buffer solution (PBS), a tris-hydrochloric acid buffer solution, a sodium citrate buffer solution, a citrate phosphate buffer solution, an acetate buffer solution, a borate buffer solution, or the like can be used.

Depending on the purpose of the present disclosure, the diluent preferably contains no impurity. However, in a case where it is difficult to obtain a diluent containing no impurity, a diluent having a low impurity concentration (for example, a diluent having an impurity concentration of less than 500 ppm) can be used.

In a case where an amount (B) of the concentrated liquid in the second tank 31 (or the third tank 32) relative to an amount (A) of the raw material liquid containing the minute useful substance (B/A) is from 1/2 to 1/200, the diluent can be supplied to the concentrated liquid in the second tank 31 (or the third tank 32). In this case, the amount (B) of the concentrated liquid may be equal to the liquid amount at the management liquid level position C set by a method described below.

Furthermore, when the roughly purified liquid is supplied from the first tank 23 to the second tank 31 (or when the raw material liquid is supplied to the second tank 31 via the flow path 43), the third control unit 130 can perform the dilution treatment. The third control unit 130 can determine that the roughly purified liquid or the raw material liquid has been supplied into the second tank 31, on the basis of the liquid level position information of the in-tank liquid amount monitoring device 35 and/or the camera. In one embodiment, the third control unit 130 performs concentration and purification by alternating tangential flow filtration until the amount of the roughly purified liquid reaches a liquid amount of from 1/2 to 1/200, and then performs the dilution treatment (or backwashing treatment) until the liquid amount reaches a set liquid amount, whereby the diluent can be supplied. In a case of performing the dilution treatment, the third control unit 130 opens the three-way valve 77A to the flow path 55B side to supply the diluent in the fourth tank 80 to the third tank 32.

The third control unit 130 can perform the backwashing treatment in a case where it is determined that the total amount of the liquids in the second tank 31 and the third tank 32 is equal to or less than a preset liquid amount (for example, equal to or less than the management liquid level position C set by the method described below). In a case of performing the backwashing treatment, the third control unit 130 opens the three-way valve 77A to the flow path 55A side. The diluent in the fourth tank 80 is supplied into the hollow fiber membrane module 33 from the third liquid inlets/outlets 33c and the 33d of the module via the flow path 55A. Thereafter, the diluent flows from the permeated liquid side to the concentrated liquid side of the hollow fiber membrane bundle 60. In a case where the hollow fiber membrane module 33 is of an internal pressure type, the diluent supplied into the module from the third liquid inlets/outlets 33c and the 33d flows from the outside to the inside of the hollow fiber membrane bundle 60. This makes it possible to recover the minute useful substance deposited on the membrane surfaces of the hollow fiber membranes, particularly on the membrane surfaces inside the hollow fiber membranes, from the first liquid inlet/outlet 33a and/or the second liquid inlet/outlet 33b into the second tank 31 and/or the third tank 32. By this treatment, the minute useful substance and the impurity deposited on the surfaces of the hollow fiber membranes are peeled off, and the permeation rate of the liquid is restored to almost the original value. In addition, the liquid is diluted with the diluent to uniformly disperse the impurities and enhance the removal efficiency. As a result, the efficiency of purifying and concentrating the liquid can be further enhanced.

Note that before performing the backwashing treatment, to prevent the permeated liquid remaining in the hollow fiber membrane module from being mixed with the backwashing liquid and flowing into the concentrated liquid side, an operation of performing a flushing treatment with a cleaning liquid (diluent) in advance and replacing the permeated liquid remaining in the hollow fiber membrane module with the cleaning liquid (diluent) may be optionally performed.

At this time, an amount of the cleaning liquid (diluent) to be used is preferably 1 to 8 times, and more preferably 2 to 6 times the volume of the permeated liquid remaining in the hollow fiber membrane module.

In a case of concentrating and purifying a certain type of extracellular vesicles in a certain type of culture supernatant, performing the flushing operation before the backwashing may reduce a residual amount of impurity proteins to half or less as compared with not performing the flushing operation.

In a case where the hollow fiber membrane module 33 is of an internal pressure type, an operation of replacing the permeated liquid remaining in the hollow fiber membrane module with a cleaning liquid (diluent) can be performed by using one of the third liquid inlets/outlets 33c and 33d for in FIG. 7 as an inlet and the other as an outlet.

The hollow fiber membrane has liquid passing resistance. Thus, when the diluent is supplied from one of the third liquid inlets/outlets 33c and 33d, the permeated liquid remaining in the hollow fiber membrane module 33 can be replaced with the diluent in a state in which leakage to the membrane side is small.

On the other hand, for example, on-off valves (not illustrated) may be provided on the flow paths in the vicinity of the outlets of the first liquid inlet/outlet 33a and the second liquid inlet/outlet 33b, and the on-off valves may be closed to stop flow of the liquid through the first liquid inlet/outlet 33a and the second liquid inlet/outlet 33b. In this state, when the diluent is supplied from one of the third liquid inlets/outlets 33c and 33d, the diluent is discharged from the other of the third liquid inlets/outlets 33c and 33d. This makes it possible to replace the permeated liquid remaining in the hollow fiber membrane module 33 with the diluent.

The timing of the flushing treatment is controlled by the third control unit. For example, description will be given on a case where on-off valves (not illustrated) are provided on flow paths near the outlets of the first liquid inlet/outlet 33a and the second liquid inlet/outlet 33b. In a case where the total of the liquid amounts in the second tank 31 and the third tank 32 is equal to or less than the value set by the second control unit, the third control unit can supply the diluent in the fourth tank 80 from any one of at least two third liquid inlets/outlets 33c and 33d of the hollow fiber membrane module 33 in a state where the on-off valves installed near the first liquid inlet/outlet 33a and the second liquid inlet/outlet 33b are closed to stop the flow of the liquid. At this time, the on-off valve 78 provided in the flow path 54 connected to the waste liquid tank is opened. Thereafter, the on-off valves installed near the first liquid inlet/outlet 33a and the second liquid inlet/outlet 33b are opened to make a state in which a liquid can flow through the first liquid inlet/outlet 33a and the second liquid inlet/outlet 33b, and the diluent in the fourth tank 80 is supplied from any one of the at least two third liquid inlets/outlets 33c and 33d of the hollow fiber membrane module 33, whereby backwashing can be performed.

At this time, the on-off valve 78 provided in the flow path 54 connected to the waste liquid tank is closed.

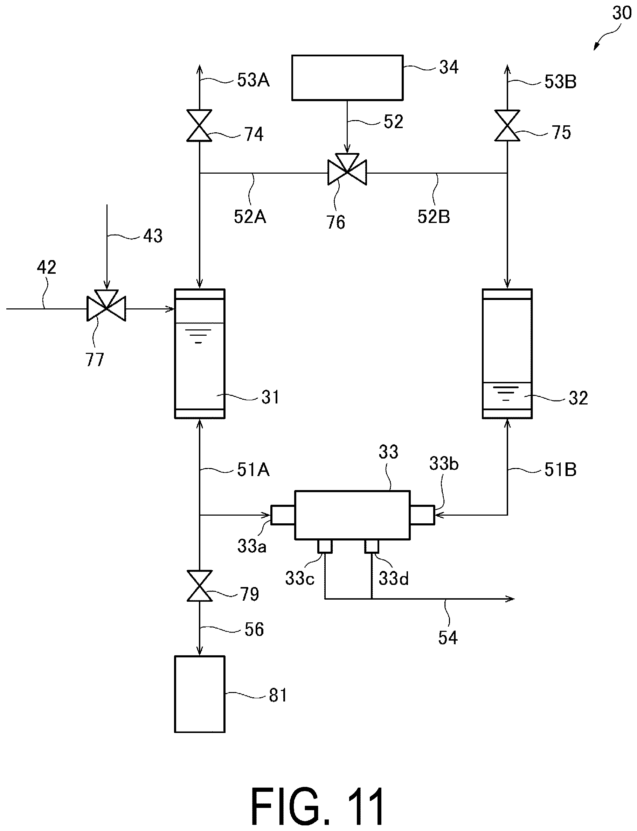

The purifying and concentrating unit 30 illustrated in FIG. 11 includes a recovery tank that recovers the concentrated liquid purified and concentrated by the hollow fiber membrane module 33. The purifying and concentrating unit 30 in FIG. 11 includes a fifth tank 81 connected to the first liquid inlet/outlet 33a of the hollow fiber membrane module 33 via a flow path 56, and a fourth control unit 140 (not illustrated) configured to control recovery of the liquids in the second tank 31 and the third tank 32 to the fifth tank 81. Note that the fifth tank 81 may be connected to the second liquid inlet/outlet 33b of the hollow fiber membrane module 33. In FIG. 11, the flow path 56 is connected to the hollow fiber membrane module 33 via the flow path 51A. While the purifying and concentrating treatment by alternating tangential flow filtration is performed in the second tank 31 and the third tank 32, an on-off valve 79 is closed. After the purifying and concentrating treatment, the on-off valve 79 is opened and the pressurizing device 34 is operated, whereby the concentrated liquid in the second tank 31 and the third tank 32 can be recovered into the fifth tank 81. Note that the tank for recovering the concentrated liquid may be disposed outside the purifying and concentrating apparatus, not inside the purifying and concentrating unit 30.

### Fourth control unit

The fourth control unit 140 controls a timing at which the concentrated liquids in the second tank 31 and the third tank 32 are recovered. An example of determination in liquid flow control of the fourth control unit 140 is illustrated in the flowchart of FIG. 16.

The fourth control unit determines liquid amounts of the concentrated liquids in the second tank 31 and the third tank 32 by the in-tank liquid amount monitoring devices 35 and 36 and/or the camera. The liquid amounts of the concentrated liquids may be determined from the management liquid level position C described below. In a case where it is determined that the purifying and concentrating treatment is completed, the fourth control unit 140 causes the pressurizing device 34 to pressurize the second tank 31 and the third tank 32. At this time, the on-off valve 74 of the gas vent path 53A, the on-off valve 75 of the gas vent path 53B, the on-off valve 78, and the three-way valves 77 and 77A are closed, and the on-off valve 79 is opened. In a case where the in-tank liquid amount monitoring devices 35 and 36 detect a change in transmittance, or in a case where it is determined from the image of the camera that the amounts of the concentrated liquids in the second tank 31 and the third tank 32 have reached 0, the fourth control unit 140 stops the pressurizing device 34 and ends the recovery of the concentrated liquid into the fifth tank 81. Specifically, the fourth control unit 140 may monitor the amount of the concentrated liquid in the flow path 56 with a fiber sensor (not illustrated) installed at the liquid inlet/outlet in the upper portion of the fifth tank 81, and may end the recovery of the concentrated liquid in a case where it is determined that the amount of the concentrated liquid has reached 0. Alternatively, the operation of recovering the concentrated liquid inside the flow path 56 can be manually operated by a person. In addition, the flow path 56 can be fixedly held by an air gripper or the like.

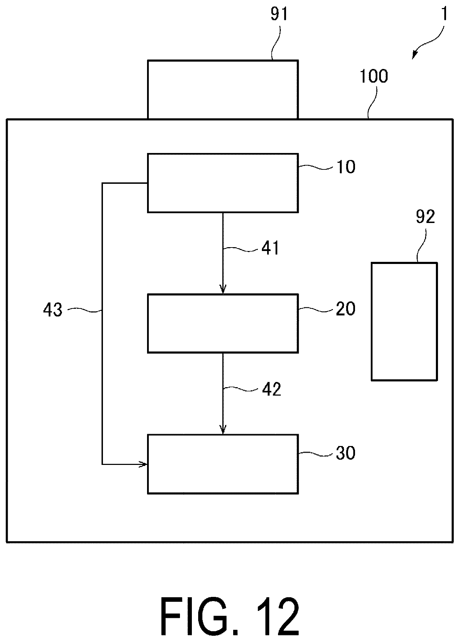

FIG. 12 illustrates a configuration example of the purifying and concentrating apparatus according to the first embodiment. In the purifying and concentrating apparatus 1 in FIG. 12, elements including the raw material supply unit 10, the removing unit 20, and the purifying and concentrating unit 30 are disposed in a housing. In the purifying and concentrating apparatus 1 in FIG. 12, the size of the housing is not particularly limited. In addition, the purifying and concentrating apparatus 1 includes a management unit (not illustrated) that manages a degree of cleanliness in the housing. This configuration can keep the inside of the purifying and concentrating apparatus 1 clean, to prevent contamination of the liquid containing the minute useful substance. Further, a fan filter device 91 that supplies filtered air into the housing is disposed on an upper portion (top portion) of the housing 100, and a UV lamp 92 is disposed in the housing 100. The combination of these can further enhance the degree of cleanliness in the housing 100.

### Fan filter device

The fan filter device 91 has a configuration capable of supplying filtered air into the housing. The fan filter device 91 may be a filter unit with a fan incorporating a pre-filter. As the pre-filter, a HEPA filter, an ULPA filter, a chemical filter, a fiber activated carbon filter, or the like can be employed depending on the degree of cleanliness in the housing 100.

### UV lamp

The UV lamp 92 is not particularly limited as long as it can emit UV (ultraviolet ray) to sterilize the inside of the housing 100. In addition, the number of the UV lamps 92 disposed in the housing 100 is not particularly limited, and two or more UV lamps may be disposed depending on the degree of cleanliness in the housing 100.

### Degree of cleanliness

In a case where the purifying and concentrating apparatus 1 has a configuration in which elements are disposed in the housing 100, the degree of cleanliness in the housing 100 can be improved by the above-described management unit. In one embodiment, the degree of cleanliness in the housing 100 may be managed to be class 8 or less, or class 5 or less in the cleanliness class defined by ISO 14644-1:2015.

### Minute useful substance

The minute useful substance is not particularly limited. In one embodiment, it may be a minute useful substance having an average particle size of less than 100 µm. In another embodiment, it may be a minute useful substance having an average molecular weight of 5000 or more. Among them, general substances useful as pharmaceuticals, cosmetics, and health foods can be employed.

Specific examples of the minute useful substance include: biomolecules such as proteins (e.g., a heat shock protein, a cytoskeletal protein, a membrane transport protein, a transmembrane protein, collagen, hyaluronic acid, chondroitin sulfate, etc.), a hormone, a cytokine, a proliferative factor, an angiogenic factor, a growth factor, an enzyme, an antibody, a plasma protein, a virus (including a virus-like particle), an extracellular vesicle (e.g., an exosome, a microvesicle, an apoptotic vesicle, etc.), a fermentum, a yeast cell, and microalgae; and polysaccharides such as fucoidan.

In addition, a specific microorganism which is a kind of biomolecule and is used for biofuels and the like is also included.

Among these, the minute useful substance may be selected from the group consisting of an extracellular vesicle, an antibody, a virus, a protein, an enzyme, and a nucleic acid.

### Method for Producing Liquid Containing Purified and Concentrated Minute Useful Substance: First Embodiment

Hereinafter, a method for producing a liquid in which the minute useful substance is purified and concentrated using the purifying and concentrating apparatus 1 according to an embodiment of the present disclosure will be described with reference to FIG. 13. The production method according to the first embodiment of the present disclosure is a method for producing the liquid in which the minute useful substance is purified and concentrated using the purifying and concentrating apparatus 1, and includes supplying a raw material liquid containing the minute useful substance to the purifying and concentrating apparatus to remove an impurity from the liquid and/or purifying and concentrating the liquid with a hollow fiber membrane.

FIG. 13 is a configuration diagram illustrating an example of the overall configuration of the purifying and concentrating apparatus 1 according to an embodiment of the present disclosure. The raw material supply unit 10 has a configuration in which the container 11 that stores a raw material liquid of the liquid containing the minute useful substance is disposed. When this apparatus is activated, the three-way valve 82 is opened, and the raw material liquid in the container 11 is supplied to a flow path 59 by falling due to its own weight and by a pump (not illustrated) provided in the flow path 41A. The raw material liquid is supplied to the flow path 41 or the flow path 43 by operating the three-way valve 82. The first control unit 110 supplies the raw material liquid to the removing unit 20 or the purifying and concentrating unit 30 on the basis of information set by the concentration of the impurity in the raw material liquid. The first control unit 110 opens the three-way valve 82 to the flow path 43 side to feed the culture solution from the flow path 43 to the purifying and concentrating unit 30.

The raw material liquid supplied to the flow path 41 is pretreated by the filtration member 22 and then supplied to the tank 24 via the flow path 41C. The liquid amount in the tank 24 can be controlled by monitoring the liquid level position with a level sensor (not illustrated). When the on-off valve 73 is opened, the pretreated liquid in the tank 24 is supplied from the flow path 41D to the flow path 41A.

### Impurity removal treatment

The pretreated liquid supplied to the carrier column 21 is made into a roughly purified liquid by adsorbing and removing an impurity from the liquid by the adsorbent in the carrier column 21, and then supplied to the first tank 23 via the flow path 42A. The impurity removal treatment by the carrier column 21 may be reducing the concentration of the impurity in the raw material liquid or the pretreated liquid to 1000 ppm or less, and preferably reducing the concentration to 500 ppm or less.

In one embodiment, the impurity removal treatment may repeat cleaning and equilibrating treatment of the carrier column 21 that has reached adsorption saturation by treatment of one batch of the raw material liquid, and further treatment of the next one batch of the raw material liquid.

The purifying and concentrating apparatus 1 in FIG. 13 includes a sixth tank 25 and a seventh tank 26. In a case of cleaning and equilibrating the carrier column 21 that has reached adsorption saturation, the on-off valve 71A (electromagnetic valve or the like) is first opened to supply the cleaning liquid in the sixth tank 25 to a filtration member 22A via the flow path 50A. After removing the impurity from the cleaning liquid by the filtration member 22A, the cleaning liquid is supplied from the flow path 41A to the carrier column 21. The cleaning liquid after cleaning the carrier column 21 can be discharged to the waste liquid tank by opening the three-way valve 72. Note that the same filtration member as the filtration member 22 can be employed as the filtration member 22A. After the cleaning treatment, the on-off valve 71B (electromagnetic valve or the like) is opened to supply the equilibrating liquid in the seventh tank 26 to a filtration member 22B. After removing the impurity from the equilibrating liquid by the filtration member 22B, the equilibrating liquid is supplied from the flow path 41A to the carrier column 21. The equilibrating liquid after equilibrating the carrier column 21 can be discharged to the waste liquid tank by opening the three-way valve 72. Note that the same member as the filtration member 22 can be employed for the filtration member 22B.

Cleaning and equilibration of the carrier column 21 can be set depending on the amount of the raw material liquid treated by the carrier column 21. In one embodiment, the cleaning and equilibration of the carrier column 21 may be performed in a case where the raw material liquid of from 5 to 100 times the volume of the carrier column 21 has been treated. For example, in a case of treating 1000 mL of the material liquid, the cleaning and equilibrating treatment of the carrier column 21 can be performed every time from 20 to 150 mL of the raw material liquid is treated by the carrier column 21, or the cleaning and equilibrating treatment can be also performed every time from 50 to 100 mL of the raw material liquid is treated.

In the production method according to an embodiment of the present disclosure, the removal of an impurity from the raw material liquid by the carrier column 21 and the purifying and concentrating of the liquid by the hollow fiber membrane of the purifying and concentrating unit 30 are preferably performed in parallel. In this case, it is possible to more efficiently produce a liquid in which the minute useful substance is purified and concentrated.

In FIG. 13, the roughly purified liquid is stored in the first tank 23 via the flow path 42A. In a case where it is determined that the amounts of the second stored liquid and the third stored liquid in the second tank 31 and the third tank 32 are the liquid amounts set in advance, the first control unit 110 sends the roughly purified liquid stored in the first tank 23 to the flow path 42B. When the direction of the three-way valve 77 is switched, the roughly purified liquid is supplied from the flow path 42B to the second tank 31. When the roughly purified liquid is supplied into the second tank 31 (or the third tank 32), the second control unit 120 (not illustrated) operates the pressurizing device 34 to start the purifying and concentrating treatment by alternating tangential flow filtration.

As described above, the purifying and concentrating apparatus 1 according to the first embodiment of the present disclosure may be configured in such a manner that the impurity removal treatment in the removing unit 20 and the purifying and concentrating treatment in the purifying and concentrating unit 30 are performed in parallel. In this case, it is preferable to arrange a plurality of tanks for each storing the liquid treated in a corresponding step. From the viewpoint of making the purifying and concentrating apparatus 1 more compact, the positional arrangement of tanks may be changed. For example, in the purifying and concentrating apparatus 1 in FIG. 13, the first tank 23 that stores the roughly purified liquid is disposed at a position above the second tank 31 and the third tank 32. However, the second tank 31 and the third tank 32 may be disposed at positions above the first tank 23. Further, as illustrated in FIG. 13, the side surfaces of the second tank 31 and the third tank 32 in the longitudinal direction may be arranged to be parallel to the longitudinal direction of the hollow fiber membrane module 33.

Hereinafter, a preferable operation in a case where the roughly purified liquid (or the raw material liquid) is subjected to alternating tangential flow filtration in the purifying and concentrating unit 30 will be described. In one embodiment, the purifying and concentrating treatment of the roughly purified liquid is preferably divided into a first treatment, a second treatment, and a third treatment. Note that before performing the first treatment, the liquid amount in the tank is set. Hereinafter, an example in which the first treatment is performed after the management liquid level position C is set will be described.

### Setting of management liquid level position C

Before performing the first treatment, an amount at the management liquid level position C is set.

The first tank 23 stores the roughly purified liquid treated by an antibody column 21. The liquid amount at the management liquid level position C is set in advance to be equal to the liquid amount at which the backwashing treatment is to be started and to be a liquid amount of from 1/2 to 1/200 of the maximum volume of the roughly purified liquid stored in the first tank 23. When the amount of the roughly purified liquid in the second tank 31 reaches the amount of the management liquid level position C set in advance, the roughly purified liquid in the first tank 23 can be supplied to the second tank 31. Further, after the roughly purified liquid is supplied to the second tank 31, the third control unit 130 opens the three-way valve 77A to the flow path 55B side to supply the diluent from the fourth tank 80 to the second tank 31.

After setting the management liquid level position C, liquid level control positions of the second tank 31 and the third tank 32 are set. The second tank 31 is pressurized by the pressurizing device 34 to feed the diluent in the third tank 32 to the second tank 31. At this time, the second control unit 120 sets a low liquid level position (L3) of the third tank 32 and supplies the diluent to the second tank 31 until the diluent in the third tank 32 reaches L3. Further, the second control unit 120 sets, as a high liquid level position (H2), the liquid level position of the second stored liquid (the mixed liquid of the roughly purified liquid and the diluent) in the second tank 31 in a case where the diluent in the third tank 32 reaches L3. In addition, from the viewpoint of preventing the second stored liquid from overflowing from the upper portion of the second tank 31, a high-high liquid level position (HH2) is set above the high liquid level position. Next, the second control unit 120 causes the pressurizing device 34 to pressurize the second tank 31, thereby feeding the second stored liquid to the third tank 32. At this time, the second control unit 120 sets a low liquid level position (L2) of the second tank 31 and supplies the second stored liquid to the third tank 32 until the second stored liquid in the second tank 31 reaches L2. Further, the second control unit 120 sets, as a high liquid level position (H3), the liquid level position of the third tank 32 in a case where the second tank 31 reaches L2. HH3 is also set for the third tank 32 from the viewpoint of preventing leakage. The second control unit 120 sets the management liquid level position C from the total liquid amounts of H2 and L3, and H3 and L2, and causes the alternating tangential flow filtration between the second tank 31 and the third tank 32 to be performed by moving the liquid within the range of the management liquid level position C. Note that L2 and L3 may be set on the basis of a change in transmittance of the liquid inlet/outlet in the tank by the in-tank liquid amount monitoring devices 35 and 36 described above, or may be set on the basis of an image of the liquid level position by the camera.

The positions of L2, L3, H2, H3, HH2, and HH3 can be appropriately set depending on the volumes of the second tank 31 and the third tank 32 and the amount of the roughly purified liquid.

### Purifying and concentrating treatment: first treatment

Next, the first treatment in the purifying and concentrating treatment will be described. The second control unit 120 causes the pressurizing device 34 to alternately pressurize the second tank 31 and the third tank 32 not to fall below the liquid amount at the management liquid level position C, thereby alternately supplying the second stored liquid and the third stored liquid to the hollow fiber membrane module 33. The concentrating and purifying operation of the liquid containing the minute useful substance is performed by performing the alternating tangential flow filtration in this manner. The permeated liquid is discharged from the third liquid inlets/outlets 33c and 33d to the waste liquid tank via the flow path 54.

When it is confirmed that the liquid amount in the second tank 31 or the third tank 32 is the set value, an appropriate amount of the roughly purified liquid can be additionally supplied to the second tank 31 or the third tank 32.

Further, when the liquid amount in the second tank 31 or the third tank 32 is equal to the liquid amount at the management liquid level position C, it is preferable to perform the backwashing treatment.

An amount of the diluent to be used in the backwashing treatment can be set to from 2 times to 200 times the liquid amount at the management liquid level position C. From the viewpoint that the volume of the second tank 31 or the third tank 32 does not become larger than necessary, the amount of the diluent is preferably from 2 to 20 times the liquid amount at the management liquid level position C. In addition, the amount of the diluent to be used in the backwashing treatment can be appropriately changed every time the backwashing operation is performed. Further, the backwashing treatment may be appropriately performed at a timing other than a case where the liquid amount in the second tank 31 or the third tank 32 is equal to the liquid amount at the management liquid level position C. Examples thereof include a case where there is a concern about a decrease in permeation rate from a situation of pressure fluctuation or flow rate fluctuation, a case where a tendency of a decrease in permeation rate can be determined, and a case where a safety operation for preventing occurrence of clogging of the hollow fiber membrane is performed.

Further, a part of the diluent may be directly fed to the second tank 31 or the third tank 32 as a dilution treatment.

An amount of the diluent to be used in the dilution treatment can be appropriately changed for each dilution operation.

In one embodiment, the second tank 31 and the third tank 32 are preferably pressurized by the pressurizing device 34 in such a manner that a linear velocity on the membrane surface of the hollow fiber membrane module 33 is from 0.3 m/sec to 2 m/sec. The "linear velocity on the membrane surface" refers to a flow rate (m³) per second of the liquid containing the minute useful substance flowing on the membrane surface of 1 m², and the unit thereof is m/sec. When the tank is pressurized by the pressurizing device 34 in such a manner that the linear velocity on the membrane surface is from 0.3 to 2.0 m/sec, the purifying and concentrating efficiency is easily improved, a pressure level for increasing the membrane surface velocity does not become too high, and a shear force applied to the minute useful substance is easily prevented from becoming too high to deform the minute useful substance. The inside of the tank may be pressurized by the pressurizing device 34 in a range in which the linear velocity on the membrane surface is from 0.5 to 1.5 m/sec.

As an operation pressure of the hollow fiber membrane module 33, a liquid inlet pressure is preferably adjusted to a range from 0.01 MPa to 0.2 MPa, more preferably from 0.02 MPa to 0.15 MPa, and still more preferably from 0.03 MPa to 0.12 MPa.

In a case where the liquid viscosity of the liquid containing the minute useful substance during operation is from 10 to 30 mPa·s at a liquid temperature of 20°C and a shear rate of 25 s⁻¹, large-diameter hollow fiber membranes having an average inner diameter of from 1.0 mm to 1.4 mm can be used in such a manner that the linear velocity on the membrane surface can be from 0.7 to 1.4 m/sec. At this time, the liquid inlet pressure is preferably adjusted to from 0.08 MPa to 0.2 MPa.

The hollow fiber membrane may be coated with an appropriate substance to improve the fouling resistance of the surface of the hollow fiber membrane and/or to increase the recovery rate of the minute useful substance. The method of the coating treatment is as described above.

In the first treatment, the purifying and concentrating treatment is preferably performed until the amount of the roughly purified liquid reaches equal to the amount at the management liquid level position C set in advance by the above-described method. After the purifying and concentrating treatment, the third control unit 130 supplies the diluent from the fourth tank 80. The diluent can be supplied until the amount thereof reaches a value of 2 times to 200 times the liquid amount at the management liquid level position C. That is, the first treatment can include diluting the concentrated liquid 2 times to 20 times.

The diluent may be directly supplied to the third tank 32 (dilution treatment), or may be supplied from the hollow fiber membrane module 33 to the second tank 31 and the third tank 32 (backwashing treatment). In a case of performing the backwashing treatment, in the purifying and concentrating apparatus 1 in FIG. 13, the three-way valve 77A is opened toward the flow path 55A side to supply the diluent from the third liquid inlets/outlets 33c and 33d of the hollow fiber membrane module 33. The diluent supplied from the hollow fiber membrane module 33 is supplied to the second tank 31 and the third tank 32. The backwashing treatment allows the concentrated liquid to be diluted while recovering the minute useful substance deposited on the membrane surface of the hollow fiber membrane, which is preferable. Hereinafter, the liquid prepared by the first treatment is referred to as a "first treated liquid".

### Purifying and concentrating treatment: second treatment

The first treated liquid is further subjected to a second treatment to prepare a liquid in which the minute useful substance is further purified and concentrated.

The impurity concentration in the first treated liquid is reduced by the first treatment described above, and thus the second treatment is preferably performed to further reduce the amount of an impurity in the first treated liquid (that is, to increase the purity of the minute useful substance). In one embodiment, in the second treatment, it is preferable that the diluent be added in an amount of from 2 times to 200 times the liquid amount at the management liquid level position C by the backwashing treatment or the dilution treatment with respect to the value set at the management liquid level position C, and then the purifying and concentrating operation be further repeated until the amount of the concentrated liquid reaches the liquid amount at the management liquid level position C. That is, the second treatment can include diluting the concentrated liquid 2 times to 20 times. As in the first treatment, the diluent may be directly supplied to the third tank 32 (dilution treatment), or may be supplied from the hollow fiber membrane module 33 to the second tank 31 and the third tank 32 (backwashing treatment). The second treatment in which the purifying and concentrating treatment and the dilution treatment are combined is preferably performed from 2 to 20 times, and more preferably from 2 to 10 times. Hereinafter, the concentrated liquid prepared by the above-described second treatment is referred to as a "second treated liquid".

### Purifying and concentrating treatment: third treatment

The second treated liquid may be further subjected to a third treatment to prepare a liquid in which the minute useful substance is further purified and concentrated. In the third treatment, no diluent is used and only the alternating tangential flow filtration operation can be performed to the extent required. Hereinafter, the concentrated liquid prepared by the above-described third treatment is referred to as a "third treated liquid".

### Recovery process

The production method according to an embodiment of the present disclosure may include recovering the second treated liquid or the third treated liquid. The second treated liquid in the second tank 31, the third tank 32, and the hollow fiber membrane module 33 can be recovered into the fifth tank 81 via the flow path 56 by being pressurized by the pressurizing device 34. When pressurization is performed by the pressurizing device 34, the fourth control unit 140 opens the on-off valve 79 and closes the on-off valves 74, 75, and 78, and the three-way valves 77 and 77A. As described above, the fourth control unit 140 may monitor the amount of the concentrated liquid in the flow path 56 by a fiber sensor installed at the liquid inlet/outlet in the upper portion of the fifth tank 81, and may end the recovery of the concentrated liquid in a case where it is determined that the amount of the concentrated liquid has reached 0. Alternatively, the operation of recovering the concentrated liquid inside the flow path 56 can be manually operated by a person. In addition, the flow path 56 can be fixedly held by an air gripper or the like.

### Flushing treatment

Before the backwashing is performed, to prevent the permeated liquid remaining in the hollow fiber membrane module from being mixed with the backwashing liquid and flowing into the concentrated liquid side, it is possible to optionally perform an operation of preliminarily perform the flushing treatment with the cleaning liquid (diluent) to replace the permeated liquid remaining in the hollow fiber membrane module with the cleaning liquid (diluent). The method of the flushing treatment is as described above.

### Operation of cleaning out minute useful substance deposited on hollow fiber membrane surface immediately before recovery treatment

The production method according to an embodiment of the present disclosure may include an operation of cleaning out the minute useful substance deposited on the hollow fiber membrane surface immediately before the recovery treatment.

As an example, in a case of concentrating and purifying a certain type of extracellular vesicles in a certain type of culture supernatant, the extracellular vesicles which are relatively firmly deposited on the surface of the hollow fiber membrane are not dispersed in the concentrated liquid, and thus the recovery rate may be low in the recovery treatment. Accordingly, an operation process of re-dispersing the extracellular vesicles deposited on the surface of the hollow fiber membrane into the concentrated liquid with a small amount of the backwashing liquid (diluent) can be performed at a timing immediately before the recovery treatment. In this case, it is preferable that the flushing operation be sufficiently performed to reduce the amount of the backwashing liquid (diluent) to be used, and the volume of the backwashing liquid (diluent) to be used is preferably, for example, from half to twice the volume of the first flow path.

The liquid flow rate at this time can be made equal to the liquid flow rate in the purifying and concentrating process.

### Other Embodiments: Stepwise Purifying and Concentrating of Liquid Containing Minute Useful Substance

Another embodiment of the production method according to the first embodiment of the present disclosure is a method for producing a purified and concentrated liquid, the method including stepwise purifying and concentrating of a liquid containing a minute useful substance. Examples of the method for performing stepwise purifying and concentrating of the liquid containing the minute useful substance include performing two or more stages of purifying and concentrating treatment using hollow fiber membrane modules having two or more average inner diameters. Specific examples thereof include a method in which a first-stage purified and concentrated liquid is produced using the hollow fiber membrane module 33 having an average inner diameter of from 0.6 mm to 1.0 mm of the present disclosure, and then a final purified and concentrated liquid is produced using a hollow fiber membrane module device having an average inner diameter of from 1.0 mm to 1.4 mm.

### Second Embodiment

Next, a second embodiment of the purifying and concentrating apparatus according to the present disclosure will be described with reference to the drawings. Note that in the following description, components having the same configurations as those of the first embodiment are denoted by the same reference numerals, and detailed description thereof is omitted.

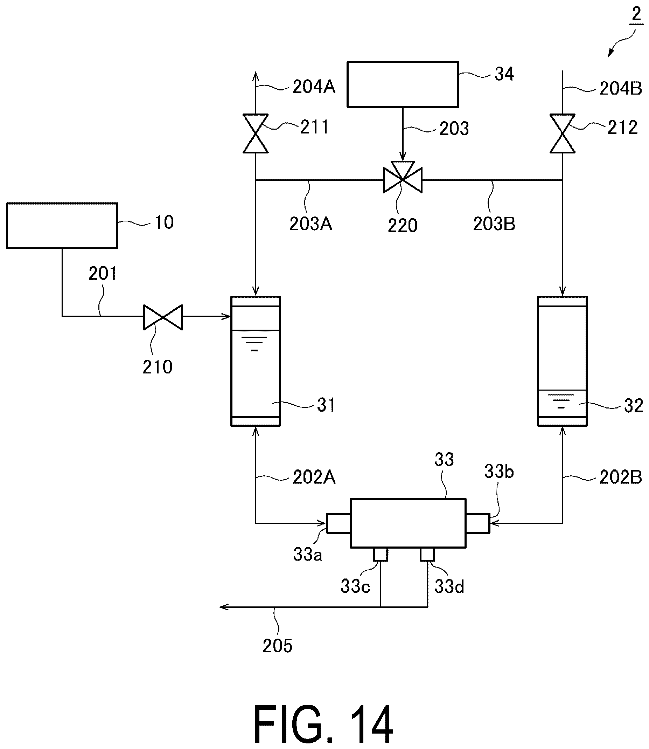

FIG. 14 is a schematic configuration diagram illustrating an example of a purifying and concentrating apparatus 2 (hereinafter, referred to as a "purifying and concentrating apparatus 2") according to the second embodiment of the present disclosure. The purifying and concentrating apparatus 2 of the present disclosure includes: a raw material supply unit 10; and a purifying and concentrating unit 30 that purifies and concentrates, with a hollow fiber membrane module 33, a liquid supplied from the raw material supply unit 10. The hollow fiber membrane module 33 has a first liquid inlet/outlet 33a and a second liquid inlet/outlet 33b for supplying the liquid to a hollow fiber membrane, and third liquid inlets/outlets 33c and 33d for discharging the liquid permeated through the hollow fiber membrane. The purifying and concentrating unit 30 includes: a second tank 31 that stores the liquid supplied from the raw material supply unit 10, the second tank 31 being connected to a flow path through which the liquid is supplied to the first liquid inlet/outlet 33a of the hollow fiber membrane module 33; a third tank 32 that stores the liquid supplied from the raw material supply unit 10, the third tank 32 being connected to a flow path through which the liquid is supplied to the second liquid inlet/outlet 33b of the hollow fiber membrane module 33; a pressurizing device 34 that selectively pressurizes either the second tank 31 or the third tank 32; a second control unit (not illustrated) that performs control in such a manner that the pressurizing device 34 selectively pressurizes an inside of the second tank 31 or an inside of the third tank 32 to alternately supply the liquid in the second tank 31 and the third tank 32 to the hollow fiber membrane module 33; and a monitoring device (not illustrated) that monitors amounts of the liquids in the second tank 31 and the third tank 32. The second control unit sets liquid amounts in the second tank 31 and the second tank 31, and causes the pressurizing device 34 to alternately pressurize the second tank 31 and the third tank 32 in such a manner that liquid amounts in the second tank 31 and the third tank 32 are the set values. The liquid amounts in the second tank 31 and the third tank 32 are determined on the basis of information by the monitoring device.

The purifying and concentrating apparatus 2 of the present disclosure may include a valve, an automatic valve, a backflow prevention valve, an orifice, a flowmeter, a pressure gauge, a liquid level gauge, a safety device such as a rupture disk, a detoxification facility, a pressure adjustment facility, an intermediate tank, a de-branching facility that can be used for operations such as sampling or multistage molecular weight division operations, and the like, which are omitted in the schematic configuration diagram.

In addition, as the monitoring device in the purifying and concentrating apparatus 2, a known liquid level meter (level gauge) of a mechanical type, a reflection type, or the like, a weight monitoring device such as a load cell, or an integrating flowmeter device can be used alone or in combination of a plurality thereof as appropriate, depending on the apparatus scale (dimension) or the like.

The purifying and concentrating apparatus 2 in FIG. 14 has a configuration in which the removing unit 20 of the purifying and concentrating apparatus 1 is not included. The purifying and concentrating apparatus 2 is also useful as an apparatus capable of purifying and concentrating a minute useful substance by automatic control. According to this configuration, the raw material liquid in the raw material supply unit 10 is directly supplied to the second tank 31 (or the third tank 32) of the purifying and concentrating unit 30 via a flow path 201. The purifying and concentrating apparatus 2 illustrated in FIG. 14 is useful as a purifying and concentrating apparatus for purifying and concentrating a highly pure raw material liquid containing a minute useful substance with a very low impurity concentration in the raw material liquid. The purifying and concentrating apparatus 1 illustrated in FIGS. 1 to 13 includes the removing unit 20 and the purifying and concentrating unit 30, and can perform the impurity removal treatment in the removing unit 20 and the purifying and concentrating treatment in the purifying and concentrating unit 30 in parallel. That is, the purifying and concentrating apparatus 1 is an apparatus capable of more efficiently preparing a concentrated liquid in which a minute useful substance is purified and concentrated. In contrast, the purifying and concentrating apparatus 2 does not include the removing unit 20, and the purifying and concentrating treatment of the raw material liquid can be performed by directly supplying the raw material liquid from the raw material supply unit 10 to the purifying and concentrating unit 30. Thus, the purifying and concentrating apparatus 2 is also useful as an apparatus for further purifying and concentrating the purified and concentrated liquid prepared by the purifying and concentrating apparatus 1.

In addition, three types of molecular weight fractionation can be performed by connecting two purifying and concentrating apparatuses 2. The wording "three types of molecular weight fractionation can be performed" means that the permeated liquid of the first membrane and the concentrated liquid of the first membrane can be sent to the second membrane to be fractionated into the permeated liquid and the concentrated liquid. The permeated liquid of the second membrane may be concentrated by the first membrane, and the permeated liquid of the first membrane may be concentrated by another membrane or another concentration apparatus. In one embodiment, the purifying and concentrating apparatus 2 can also be used as an apparatus for fractionating the purified and concentrated liquid prepared by the purifying and concentrating apparatus 1 into a low molecular weight (for example, a minute useful substance having a molecular weight cutoff of 10000 to 100000) and a high molecular weight (a minute useful substance having a molecular weight cutoff of more than 100000).

In the purifying and concentrating apparatus 2 in FIG. 14, the second tank 31 and the third tank 32 may have the same configuration as in the purifying and concentrating apparatus 1. It is assumed that the purifying and concentrating apparatus 2 uses, for example, a concentrated liquid prepared by an apparatus such as the purifying and concentrating apparatus 1 as a raw material liquid. Thus, volumes of the second tank 31 and the third tank 32 can be made smaller than those of the second tank 31 and the third tank 32 of the purifying and concentrating apparatus 1. The tanks can also be larger than those of the purifying and concentrating apparatus 1. It is also possible to omit the first tank 23. Further, similarly to the purifying and concentrating apparatus 1, in a case where the liquid level of the transparent portion of the liquid inlet/outlet of the second tank 31 or the third tank 32 is managed by a fiber sensor or the like, the second tank 31 and the third tank 32 may be made of an opaque material such as a metal or a fiber-reinforced resin.

In the purifying and concentrating apparatus 2 in FIG. 14, a liquid transfer device is not limited to the pressurizing device 34, but may be a liquid feeder that performs pump liquid feeding and/or liquid feeding using potential energy alone or in combination.

To use potential energy for liquid transfer, it is also possible to control the heights of the liquid levels in the second tank 31 and the third tank 32 using a lifting device or the like, or to appropriately change the height position of the liquid inlet/outlet.

In the purifying and concentrating apparatus 2 in FIG. 14, to suppress foaming of the liquid in the second tank 31 or the third tank 32, in a case where the height position of the liquid inlet is higher than the liquid surface, it is possible to appropriately adjust a pipe diameter and a liquid feed flow rate not to involve air in an upper portion of the tank, and to install equipment capable of automatically adjusting the height from the pipe outlet to the liquid surface, foam breaking/defoaming equipment, or the like. In some cases, an appropriate defoaming agent can be added.

In the purifying and concentrating apparatus 2 in FIG. 14, the hollow fiber membrane in the hollow fiber membrane module 33 may be coated with an appropriate substance in the same manner as in the purifying and concentrating apparatus 1 to improve the fouling resistance of the surfaces of the hollow fiber membrane and/or to increase the purifying and concentrating efficiency and the recovery rate of extracellular vesicles.

In the purifying and concentrating apparatus 2 in FIG. 14, the average inner diameter of the hollow fiber membrane in the hollow fiber membrane module 33 can be appropriately adjusted depending on the viscosity of the liquid. In one embodiment, the average inner diameter of the hollow fiber membrane may be from 0.2 to 2.0 mm. Similarly to the above-described purifying and concentrating apparatus 1, in a case where the liquid viscosity is high (for example, in a case where the liquid viscosity at a liquid temperature of 20°C and a shear rate of 25 s⁻¹ is from 10 to 30 mPa·s), it is preferable to use a large-diameter hollow fiber membrane having an average inner diameter of from 1.0 mm to 1.4 mm. In a case where the liquid viscosity of the liquid containing the minute useful substance during operation is low (for example, in a case where the liquid viscosity at a liquid temperature of 20°C and a shear rate of 25 s⁻¹ is less than 10 mPa·s), it is preferable to use a hollow fiber membrane having an average inner diameter of from 0.2 mm to 1.0 mm. Note that when the average inner diameter of the hollow fiber membranes increases, an effective membrane area tends to decrease in a case of using the hollow fiber membranes as a hollow fiber membrane module. Accordingly, it is preferable to use hollow fiber membranes having an average inner diameter of from 0.2 mm to 1.0 mm from the viewpoint of easily suppressing a decrease in effective membrane area in a case of being formed into the hollow fiber membrane module.

The purifying and concentrating apparatus 2 in FIG. 14 may have the same elements as those of the purifying and concentrating apparatus 1 in FIGS. 1 to 13 in addition to the illustrated elements. For example, in the purifying and concentrating apparatus 2 in FIG. 14, the monitoring device that monitors the liquid amounts in the second tank 31 and the third tank 32 may include the in-tank liquid amount monitoring devices 35 and 36 and/or the camera described above. The second control unit of the purifying and concentrating apparatus 2 can also have the same configuration as that of the second control unit 120 of the purifying and concentrating apparatus 1. That is, the second control unit can determine the tank to be pressurized by the pressurizing device 34 by grasping the liquid amounts in the tanks on the basis of the change in transmittance of light detected by the optical sensors included in the in-tank liquid amount monitoring devices 35 and 36. The second control unit can also determine the tank to be pressurized by the pressurizing device 34 by grasping the liquid amounts in the tanks from information of the liquid level position output from the camera. In the purifying and concentrating apparatus 2, the monitoring device preferably includes the in-tank liquid amount monitoring devices 35 and 36.

As described above, in the purifying and concentrating apparatus 2, the liquid transfer device is not limited to the pressurizing device, and pump liquid feeding and/or liquid feeding using potential energy can be performed alone or in combination. In this case, the second control unit can be configured to control the heights of the liquid levels in the second tank 31 and the third tank 32 using a lifting device or the like, and can also be configured to control the position of the height of the liquid inlet/outlet. With such a configuration, the second control unit can control the liquid feeder in such a manner that the liquid in the second tank and the liquid in the third tank is selectively supplied to the hollow fiber membrane module.

The purifying and concentrating unit 30 in the purifying and concentrating apparatus 2 in FIG. 14 can include a cleaning tank that supplies a diluent to the hollow fiber membrane module 33 to clean a plurality of hollow fiber membranes in the hollow fiber membrane bundle 60. That is, the purifying and concentrating unit 30 of the purifying and concentrating apparatus 2 may include a fourth tank (reference numeral 80 in FIG. 10) that stores a diluent, the fourth tank being connected to a flow path through which the diluent is supplied from the third liquid inlets/outlets 33c and 33d of the hollow fiber membrane module 33. In addition, the same third control unit 130 as that of the purifying and concentrating apparatus 1 may be provided. That is, in a case where the total of the liquid amount in the second tank 31 and the liquid amount in the third tank 32 is equal to or less than the value set by the second control unit, the third control unit in the purifying and concentrating apparatus 2 can supply the diluent in the fourth tank from the third liquid inlets/outlets 33c and 33d of the hollow fiber membrane module 33. According to this configuration, the raw material liquid can be purified and concentrated while recovering the minute useful substance deposited on the membrane surfaces inside the hollow fiber membranes. Further, the fourth tank may be connected to the second tank 31 or the third tank 32.

In the purifying and concentrating apparatus 2, as in the purifying and concentrating apparatus 1, before supplying the diluent to the hollow fiber membrane module 33 (backwashing), to prevent the permeated liquid remaining in the hollow fiber membrane module 33 from being mixed with the backwashing liquid and flowing into the concentrated liquid side, the operation of flushing with a cleaning liquid (diluent) in advance and replacing the permeated liquid remaining in the hollow fiber membrane module with the cleaning liquid (diluent) can be optionally performed.

The purifying and concentrating unit 30 in the purifying and concentrating apparatus 2 in FIG. 14 may include a fifth tank 81 for recovering the concentrated liquid in the second tank 31, the third tank 32, and the hollow fiber membrane module 33. In addition, a fourth control unit that controls the recovery of the concentrated liquid to the fifth tank 81 may be provided. The configurations of the fifth tank 81 and the fourth control unit may be the same as those of the purifying and concentrating apparatus 1.

The minute useful substance purified and concentrated by the purifying and concentrating apparatus according to the second embodiment may be the same as or different from that described in the purifying and concentrating apparatus 1. The minute useful substance to be purified and concentrated by the purifying and concentrating apparatus according to the second embodiment can include minute useful substances in industrial applications such as an electroactive polymer, a rare metal colloidal particle, a pigment, a dye, and an inorganic or organic nanoparticle. In general, in a case where a liquid containing these minute useful substances in industrial applications is subjected to the purifying and concentrating treatment, treatment for removing an impurity from the liquid is not required. Thus, the apparatus according to the second embodiment of the present disclosure can be suitably used.

### Method for Producing Liquid Containing Purified and Concentrated Minute Useful Substance: Second Embodiment

Examples of the method for producing a liquid in which a minute useful substance is purified and concentrated using the purifying and concentrating apparatus 2 of the present disclosure include a method including a process in which the impurity removal treatment is omitted in the production method using the above-described purifying and concentrating apparatus 1. Note that the purifying and concentrating apparatus 2 does not include the removing unit 20, but may include a pretreatment process such as pre-filtration treatment using a microfiltration membrane or the like or coagulation and sedimentation treatment.

In the purifying and concentrating apparatus 2 in FIG. 14, when an on-off valve 210 is opened after the raw material liquid is charged into the container or the like in the raw material supply unit 10, the raw material liquid is supplied to the second tank 31 (or the third tank 32) via the flow path 201. The raw material liquid supplied to the second tank 31 (or the third tank 32) is purified and concentrated by alternating tangential flow filtration. After the purifying and concentrating treatment, the concentrated liquid in the tank and the hollow fiber membrane module is recovered to the fifth tank 81.

### Other Embodiments: Molecular Weight Fractionation and Purifying and Concentrating of Minute Useful Substance

Another embodiment in the production method according to the second embodiment of the present disclosure is a method for producing a purified and concentrated liquid including molecular weight fractionation and purifying and concentrating treatment of a minute useful substance. Examples of the method of performing molecular weight fractionation of a minute useful substance as two or more components and concentration and purification of each liquid include connecting two or more purifying and concentrating apparatuses 2 of the present disclosure including hollow fiber membrane modules having two or more molecular weight cutoffs to each other, and performing the purifying and concentrating treatment in two or more stages.

Specifically, in a case where a purified and concentrated liquid of a liquid containing chondroitin sulfate having a wide molecular weight distribution is produced, an apparatus having a molecular weight cutoff of 10000 and an apparatus having a molecular weight cutoff of 70000 are connected to each other, or the purifying and concentrating operation is performed in two or more stages, whereby a purified (molecular-weight purified) and concentrated liquids of chondroitin sulfate having three molecular weight distributions can be prepared. In this case, the concentration of the component having a molecular weight cutoff of less than 10000 may be performed by concentrating the permeated liquid from the apparatus having a molecular weight cutoff of 10000 using an apparatus other than a membrane separation apparatus, or using an NF membrane concentration apparatus (for example, an apparatus using a membrane described in Japanese Patent No. 7021400).

### Examples

Hereinafter, the present invention will be described in detail showing examples, but the present invention is not limited to the following examples.

### Preparation of hollow fiber membrane module 33

A hollow fiber membrane bundle 60 formed by bundling 75 hollow fiber membranes (available from Daicen Membrane Systems Ltd.) made of cellulose acetate (CA) and having an average inner diameter of 0.8 mm, an average outer diameter of 1.3 mm, and a molecular weight cutoff of 300000 was accommodated in a cylindrical container 61 made of polycarbonate to prepare an internal pressure type hollow fiber membrane module illustrated in FIG. 7. The pure water permeation flow rate at 0.1 MPa of the hollow fiber membrane module 33 was 28.8 L/Hr. The γ-globulin permeability measured by the following measurement method was 54%, the effective length of the hollow fiber membrane was 17 cm, the effective membrane area was 0.0312 m², and the filling rate of the hollow fiber membranes was 30%.

### γ-Globulin permeability measurement

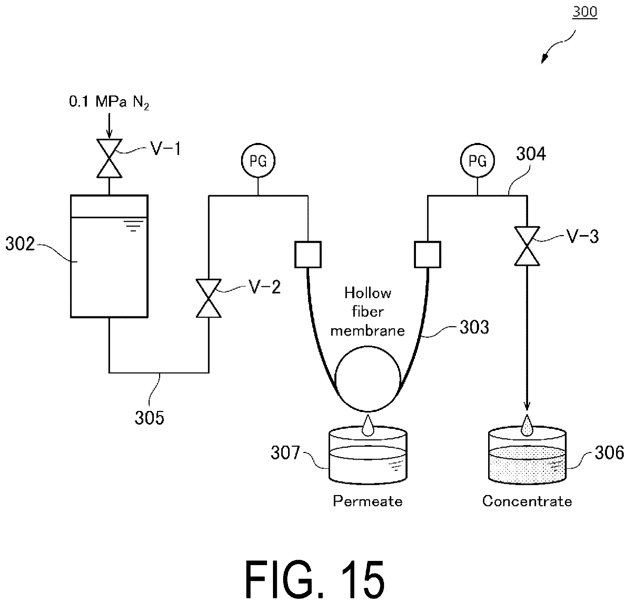

The γ-globulin permeability was measured by the following operation using a γ-globulin permeability measuring apparatus 300 illustrated in FIG. 15.
1) An aqueous γ-globulin solution (γ-globulin concentration: 100 ppm) was charged into a tank 302.
2) A hollow fiber membrane 303 was connected to a flow path 305.
3) Nitrogen gas was injected through a valve V-1 to pressurize the tank 302 at 0.1 MPa. The aqueous solution was filtered from the inner surface toward the outer surface of the hollow fiber membrane 303, the filtrate was discarded into a container 307, and the permeated liquid was recovered into a container 306. Note that the initial permeated liquid (about 100 mL) was discarded, and the permeated liquid discharged thereafter was used as a sample for measuring the permeability.

After preparing the sample of the permeated liquid in the apparatus 300, the γ-globulin permeability was measured in the following method.

4) A spectrophotometer (available from Shimadzu Corporation, product name "UV2450") was used to measure an absorbance (A1) at 280 nm of the aqueous γ-globulin solution (100 ppm) and an absorbance (A2) at 280 nm of the permeated liquid, and the γ-globulin permeability (%) was calculated by a calculation formula (A2/A1 × 100).

### Purifying and concentrating apparatus 1

A specific operation in a case of using the purifying and concentrating apparatus 1 in FIG. 13 will be described below.

Elements including the raw material supply unit 10, the removing unit 20, and the purifying and concentrating unit 30 are disposed in a housing 100 (not illustrated) having a width of 800 mm, a depth of 350 mm, and a height of 1745 mm.

A fan filter device 91 (available from AS ONE CORPORATION, product name "PURE SPACE 01", equipped with an ULPA filter unit, air flow rate of 1 m³/min) (not illustrated) is disposed at the top of the purifying and concentrating apparatus 1, whereby filtered air flows down into the housing 100.

Four UV lamps 92 (glow type germicidal lamps available from Daiwa Electric Co., Ltd.) (not illustrated) are mounted in the housing 100.

A movable caster with an adjustable stopper (not illustrated) is attached to the bottom of the purifying and concentrating apparatus 1.

A door (not illustrated) is disposed on the front surface of the purifying and concentrating apparatus 1, and a touch screen (available from Keyence Corporation, product name "Touch Panel Display Model: VT-W4M") (not illustrated) for managing processes of the purifying and concentrating apparatus 1 is attached to the door.

A compressed air intake (not illustrated) and a DC power supply (not illustrated) are disposed in the purifying and concentrating apparatus 1. Compressed air is reduced in pressure to 0.05 MPa by a regulator, and performs switching of the liquid supply destination and supply control via a plurality of on-off valves (two-port electromagnetic valves), a three-way valve (three-port electromagnetic valve), and an electromagnetic valve manifold, for cleaning, equilibrating treatment, impurity removal treatment, purifying and concentrating treatment, and backwashing treatment of the carrier column 21. The compressed air decompressed to 0.3 MPa is also used for an air grip when the concentrated liquid is recovered.

### Raw material supply unit 10

A container 11 that stores a raw material liquid is disposed.

### Removing unit 20

The carrier column 21 has a configuration in which 5 mL of an adsorbent carrier (available from Cytiva, product name "Capto (trade name) Core 700" (17548101)) is enclosed in a column having an inner diameter of 25 mmφ.

As the filtration member 22, a microfiltration membrane (available from Millipore Corporation, product name "Millex-GP", material: polyether sulfone, pore diameter: 0.22 µm) is used.

A 2 M sodium chloride aqueous solution as a cleaning liquid is charged into the sixth tank 25.

50 mM Tris-HCl (pH 7.2) as an equilibrating liquid is charged into the seventh tank 26.

As illustrated in FIG. 13, the liquid is supplied from the tank 24, the sixth tank 25, and the seventh tank 26 to the carrier column 21 through the flow path 41A via a plurality of on-off valves (electromagnetic valves). A tube pump (not illustrated) is disposed on the flow path 41A.

### Purifying and concentrating unit 30

The hollow fiber membrane module 33 produced as described above is used as the hollow fiber membrane.

A transparent container (volume of 120 mL) made of poly(meth)acrylate was used as each of the second tank 31 and the third tank 32. In-tank liquid amount monitoring devices 35 and 36 (pipe attachment type liquid level detection fiber unit (model: FU-95S) available from Keyence Corporation) each including an optical sensor are disposed in the liquid inlets/outlets of the bottom portions of the second tank 31 and the second tank 31.

A phosphate buffer solution (PBS) as a diluent is charged into the fourth tank 80.

### Example 1

As the raw material liquid, 800 mL of a culture supernatant of mesenchymal stem cells containing exosomes prepared by the following method is charged into the container 11.

### Preparation of culture supernatant of mesenchymal stem cells containing exosome

Human adipose tissue-derived mesenchymal stem cells were cultured using an Ultra ExoM Culture Medium for Extracellular Vesicles (FK-K0204024 available from Santeja Inc.).

In a state where the cells cover about 80% of an adhesion surface of the culture container, the culture medium was replaced with the Ultra ExoM Culture Medium containing no phenol red, and the mesenchymal stem cells are cultured for 48 hours.

Subsequently, the culture supernatant is centrifuged at a centrifugal force of 2000 × g for 10 minutes at 4°C to remove cell debris thereby preparing a culture supernatant.

The raw material liquid in the container 11 is supplied to the filtration member 22 to be wholly pretreated, and then stored in the tank 24 (pretreated liquid storage tank).

Next, 50 mL of the cleaning liquid is supplied from the sixth tank 25 (cleaning liquid storage tank) to the carrier column 21 at 5 mL/min to clean the carrier column 21, and then the cleaning liquid is discharged into the waste liquid tank. Thereafter, 50 mL of the equilibrating liquid is supplied from the seventh tank 26 (equilibrating liquid storage tank) to the carrier column 21 at 5 mL/min to equilibrate the carrier column 21, and then the equilibrating liquid is discharged into the waste liquid tank.

### Impurity removal treatment

50 mL of the pretreated liquid in the tank 24 is supplied to the carrier column 21, and host-derived proteins (HCPs), DNAs, and the like in the pretreated liquid are adsorbed and removed by the carrier. The resulting roughly purified liquid is supplied to the first tank 23 via the flow path 42A. Thereafter, the second cleaning and equilibrating treatment of the carrier column 21 is performed under the same conditions as described above. After the second cleaning and equilibrating treatment, 50 mL of the pretreated liquid in the tank 24 is supplied to the carrier column 21 to perform the second impurity removal treatment. Thereafter, the third cleaning and equilibrating treatment of the carrier column 21 and the third impurity removal treatment are performed in the same manner. Subsequently, the fourth to sixteenth cleaning and equilibrating treatments of the carrier column 21 and the fourth to sixteenth impurity removal treatments are performed.

### Purifying and concentrating treatment

The impurity removal treatment and the purifying and concentrating treatment are performed in parallel.

First, the roughly purified liquid (about 200 mL) in the first tank 23 stored by the first to fourth impurity removal treatments is fed to the second tank 31 with compressed air of 0.05 MPa. At this time, after the first roughly purified liquid (about 50 mL) is fed to the second tank 31, the second tank 31 is pressurized by the pressurizing device 34 to set the liquid amount at the management liquid level position C to 10 mL, and the purifying and concentrating treatment by alternating tangential flow filtration (TFF) is performed in such a manner that the liquid amount in the second tank 31 does not fall below 10 mL. Thereafter, while sequentially feeding the second to fourth roughly purified liquids to the second tank 31, the purifying and concentrating treatment by the alternating tangential flow filtration (TFF) is continuously performed in such a manner that the liquid amount of the second tank 31 does not fall below 10 mL.

Next, backwashing treatment is performed. For the backwashing treatment, 90 mL of a diluent is used. A phosphate buffer solution (PSB) is used as the diluent. After the backwashing treatment is performed with the diluent, the purifying and concentrating treatment by the alternating tangential flow filtration (TFF) is performed in such a manner that the liquid amount in the second tank 31 does not fall below 10 mL. Thereafter, the purifying and concentrating treatment and the backwashing treatment are performed while the roughly purified liquids produced by the fifth to eighth impurity removal treatments, the ninth to twelfth roughly purified liquids, and the thirteenth to sixteenth roughly purified liquids are added to the second tank 31. Note that the backwashing treatment is performed every time the feed amount of the roughly purified liquid after the backwashing treatment reaches 200 mL, and the purifying and concentrating treatment is performed by the alternating tangential flow filtration (TFF) in such a manner that the liquid amount in the second tank 31 does not fall below 10 mL. After the purifying and concentrating treatment and the backwashing treatment of a total of 800 mL of the roughly purified liquid are performed and the liquid amount in the second tank 31 reaches 10 mL, the backwashing treatment and the purifying and concentrating treatment are repeated three times with the diluent amount set to 90 mL (10-fold dilution) to prepare a purified and concentrated liquid of exosomes.

### Recovery treatment

The entire amount of the resulting purified and concentrated liquid of exosomes is recovered in the fifth tank 81 via the flow path 56. In the recovery treatment of the concentrated liquid, the amount of the concentrated liquid in the flow path 56 is monitored by a fiber sensor installed at the liquid inlet/outlet in the upper portion of the fifth tank 81, and the concentrated liquid in the two tanks is recovered into the fifth tank 81 until the fiber sensor detects air bubbles. The yield of the purified and concentrated exosome liquid produced at this time is from 0.8 to 80 mL, and the exosome recovery rate is from 50 to 100%. In addition, a protein removal rate is expected to be 80% or more.

### Example 2

As the raw material liquid, 800 mL of a culture supernatant of human adipose tissue-derived mesenchymal stem cells was used. When a protein concentration and CD9/CD63 positive extracellular vesicles in the culture supernatant were quantitatively analyzed by the Bradford method and the ELISA method, respectively, the protein concentration was 0.49 mg/mL and a concentration of the CD9/CD63 positive extracellular vesicles was 152 pg/mL.

80 mL of a mixed-mode carrier having cation exchange and size exclusion equilibrated with 50 mM of Tris-HCl (pH 7.2) was added to the above culture supernatant, and the mixture was dispensed into a 50 mL tube and then mixed by inversion at room temperature for 10 minutes at a rotational speed of 10 rpm/min using a rotator. Thereafter, the mixture was centrifuged at room temperature for 5 minutes at room temperature at 10000 xg, and the supernatant was further filtered using a 0.22 µm filtration unit to remove the carrier. 790 mL of the resulting carrier-treated culture supernatant had a protein concentration of 0.06 mg/mL and an exosome concentration of 144 pg/mL.

Next, the carrier-treated culture supernatant was supplied from the container 11 to the second tank 31 via the flow path 43, and subjected to alternating tangential flow filtration under the pressurization condition of the pressurizing device 34 set to a nitrogen gas pressure of 0.04 MPa. When the remaining amount became 10 mL, 90 mL of the phosphate buffer solution (PBS) was supplied from the fourth tank 80 to the hollow fiber membrane module 33 side to perform backwashing. Thereafter, the alternating tangential flow filtration and the supply of 90 mL of the phosphate buffer solution were further repeated twice, and then the alternating tangential flow filtration was performed to prepare 12.9 mL of a purified and concentrated liquid. The resulting purified and concentrated liquid had a protein concentration of 0.09 mg/mL, and an exosome concentration of 7149 pg/mL. The protein removal rate calculated from the concentration and the liquid amount was 99% or more, and the recovery rate was 76%.

### Comparative Example 1

As Comparative Example 1, 800 mL of a culture supernatant having a protein concentration of 0.52 mg/mL and an exosome concentration of 130 pg/mL was used as the raw material liquid. The raw material liquid was supplied from the container 11 to the second tank 31 via the flow path 43, and subjected to the purifying and concentrating treatment by alternating tangential flow filtration. Note that in Comparative Example 1, the purifying and concentrating treatment was performed under the same conditions as in Example 2 except that the cleaning process in the middle was performed by forward washing instead of backwashing. The forward washing was performed by supplying the diluent in the fourth tank 80 to the third tank 32 via the flow path 55B, and then supplying the diluent to the hollow fiber membrane module 33 to clean the hollow fiber membranes. Finally, 11 mL of a purified and concentrated liquid was produced. The purified and concentrated liquid had a protein concentration of 0.02 mg/mL or less, and an exosome concentration of 2595 pg/mL. The protein removal rate calculated from the concentration and the liquid amount was 99% or more, and the recovery rate was 27%. From this result, it has been found that the recovery rate is improved by performing the backwashing.

### Example 3

Here, a method for further improving the recovery rate of the minute useful substance by coating the surface of the hollow fiber membrane with a biomolecule in the purifying and concentrating process was examined.

As the raw material liquid, 790 mL of a culture supernatant of mesenchymal stem cells having a protein concentration of 0.49 mg/mL and an exosome concentration of 166.4 pg/mL was used.

The concentration of exosomes was an amount corresponding to a CD9/CD63 fusion standard protein used for preparing the calibration curve when quantitative analysis was performed by a CD9/CD63 ELISA method (EXH0102EL, Cosmo Bio Co., Ltd.).

To 790 mL of the culture supernatant, 79 mL of a carrier equilibrated with 50 mM Tris-HCl (pH 7.2) (a porous granular material in which the insides of pores are positively charged, an adsorbent capable of adsorbing and holding negatively charged unnecessary substances except extracellular vesicles) was added, and mixed by inversion at room temperature for 10 minutes using a rotator. Subsequently, the carrier was removed from the carrier-cell supernatant mixture using a 0.22 µm filtration unit (S2GPU11RE, Merck Millipore) to prepare a carrier-treated culture supernatant.

Next, the carrier-treated culture supernatant was purified and concentrated by filtration in an alternating tangential flow scheme at a filtration pressure of from 30 to 40 kPa. As the filtration membrane, a cellulose acetate hollow fiber membrane module (molecular weight cutoff of 300000, membrane area of 0.062 m²) was used. The filtration membrane having a membrane surface coated with a biomolecule was used. The coating treatment was performed by feeding 1.3 mg/mL of a skim milk solution dissolved in PBS through the filtration membrane at a liquid flow rate of 200 mL/min for 10 minutes. As a result, a coated membrane was produced in which a certain type of biomolecule in skim milk was adsorbed and bonded as a thin layer to the membrane surface. The filter membrane was then rinsed with 250 mL of PBS to remove excess skim milk just deposited on the thin layer.

Here, the bonding state between the filtration membrane and the certain type of biomolecule in the skim milk was strengthen to such an extent that the biomolecule did not fall off under washing with water and operation conditions, and that the filtration performance hardly decreased even in a long-term operation.

The alternating tangential flow filtration was repeated until the liquid amount of the concentrated liquid became 10 mL, and then 90 mL of PBS was supplied to the hollow fiber membrane module to dilute the concentrated liquid. These concentration-filtration, and dilution processes were further repeated twice, followed by alternating tangential flow filtration to produce 11.9 mL of a purified and concentrated liquid (purified extracellular vesicles). The extracellular vesicles in the finally resulting concentrated liquid of exosomes were quantitatively analyzed by the CD9/CD63 ELISA method (EXH0102EL, Cosmo Bio Co., Ltd.). As a result, the concentration was 3746.7 pg/mL corresponding to the CD9/CD63 fusion standard protein used for preparing the calibration curve, and the recovery rate was 33.9%.

### Comparative Example 2

When the same operation was performed using a hollow fiber membrane not coated with skim milk, the recovery rate was 7.4%.

From the results of Example 3 and Comparative Example 2, it has been found that the recovery rate of the minute useful substance is improved by using the hollow fiber membrane coated with a biomolecule (that is, the hollow fiber membrane in which a biomolecule of a type different from the minute useful substance is attached to at least a partial region).

### Example 4

Here, a method for further improving the purity of the minute useful substance in the purified and concentrated liquid prepared in the purifying and concentrating process was examined.

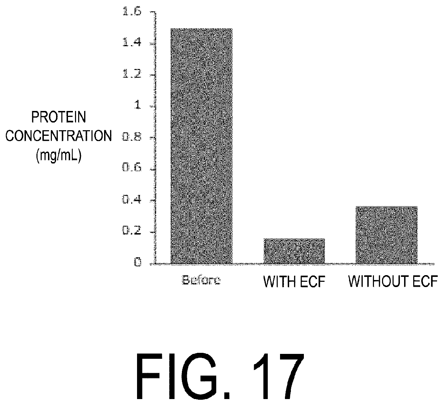

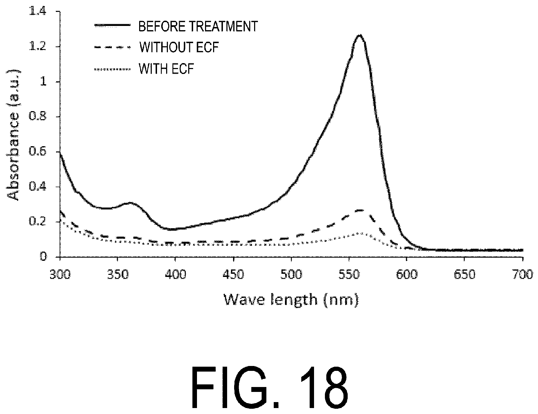

As the raw material liquid, 40 mL of a culture supernatant of amnion-derived mesenchymal stem cells having a protein concentration of 1.49 mg/mL and an exosome concentration of 196 pg/mL was used. The exosomes in the culture supernatant were separated, purified, and concentrated by the alternating tangential flow scheme at a filtration pressure of from 30 to 40 kPa using a purifying and concentrating apparatus equipped with the hollow fiber membrane module 33 made of cellulose acetate (molecular weight cutoff of 300000, membrane area of 0.01 m², permeation-side volume (i.e., extracapillary space (ECS) of 10 mL). After the culture supernatant was concentrated to about 10 mL, 40 mL of the diluent (PBS) was injected from the third liquid inlet/outlet 33c of the hollow fiber membrane module and discharged to the third liquid inlet/outlet 33d. This flushed the impurity in the permeated liquid retaining in the ECS with PBS. Thereafter, 90 mL of the same diluent (PBS) was injected under pressure from the permeation side to the concentration side of the hollow fiber membrane module 33 to perform back pressure washing (backwashing). Separation and purification operations were performed again under the same conditions, and when the culture supernatant reached about 10 mL, similar flushing and backwashing were further performed twice. After backwashing was performed three times, the culture supernatant was concentrated to 5.5 mL. When the residual amount of protein in the resulting purified and concentrated liquid was measured by BCA assay, the protein concentration was 0.16 mg/mL. The results are shown in FIG. 17. Further, an absorbance of the resulting purified and concentrated liquid was measured using an absorption microplate reader Infinite M Nano+ available from Tecan Trading AG. The results are shown in FIG. 18.

### Comparative Example 3

In the same separation and purification operations as in Example 4, the culture supernatant was concentrated to 5.2 mL without flushing the ECS with PBS. When the residual amount of protein in the resulting purified and concentrated liquid was measured by BCA assay, the protein concentration was 0.36 mg/mL. The results are shown in FIG. 17. In addition, an absorbance of the resulting purified and concentrated liquid was measured in the same manner as in Example 4. The results are shown in FIG. 18.

In FIGS. 17 and 18, "BEFORE" represents a sample before the purifying and concentrating treatment, "WITH ECF" represents the purified and concentrated liquid prepared by the method of Example 4, and "WITHOUT ECS" represents the purified and concentrated liquid prepared by the method of Comparative Example 3. As is clear from FIG. 17, according to the method of Example 4, the concentration of protein as an impurity is further reduced, and the purity of exosomes can be further improved. In FIG. 18, the peak near the wavelength of 557 nm is a peak derived from phenol red contained as a pH indicator in the culture solution and corresponds to an impurity in the purified and concentrated liquid. As shown in FIG. 18, according to the method of Example 4, the concentration of phenol red as an impurity is further reduced, and the purity of exosomes can be further improved.

From these results, it has been found that when the inside of the ECS is washed away (flushed) with a diluent such as PBS or physiological saline before the backwashing process of the hollow fiber membrane module to remove the impurity remaining in the ECS, the concentration of the impurity in the resulting purified and concentrated liquid can be further reduced, and as a result, the purity of the minute useful substance can be further improved. As a non-limiting reason, it is considered that flushing can prevent an impurity in the ECS from re-entering the purified and concentrated liquid during the subsequent backwashing process with the diluent.

### Example 5

As the raw material liquid, 200 mL of a culture supernatant of amnion-derived mesenchymal stem cells having a protein concentration of 1.25 mg/mL and an exosome concentration of 138 pg/mL was used. Exosomes in the culture supernatant were separated, purified, and concentrated by the alternating tangential flow scheme using a purifying and concentrating apparatus equipped with a hollow fiber membrane module made of cellulose acetate (molecular weight cutoff of 300000, membrane area of 0.01 m², ESC of 10 mL). After the culture supernatant was concentrated to about 17 mL, 40 mL of the diluent (PBS) was injected from the third liquid inlet/outlet 33c of the hollow fiber membrane module and discharged to the third liquid inlet/outlet 33d. This flushed the impurity in the permeated liquid retaining in the ECS with PBS. Thereafter, 160 mL of the same diluent (PBS) was injected under pressure from the permeation side to the concentration side of the hollow fiber membrane module 33 to perform back pressure washing (backwashing). Separation and purification operations were performed again under the same conditions, and when the culture supernatant reached about 17 mL, similar flushing and backwashing were further performed twice. The culture supernatant after backwashing was concentrated to 17 mL. Backwashing was performed again to wash out minute useful substances adhering to the surfaces of the hollow fiber membranes into the purified and concentrated liquid, and 50 mL of the purified and concentrated liquid was recovered. The extracellular vesicles in the resulting purified and concentrated liquid were quantitatively analyzed by the CD9/CD63 ELISA method (EXH0102EL, Cosmo Bio Co., Ltd.) to find that the concentration was 486 pg/mL corresponding to the CD9/CD63 fusion standard protein used for preparing the calibration curve, and the recovery rate was 88%. From this result, it has been found that when the diluent is further supplied to the hollow fiber membrane module immediately before recovering the purified and concentrated liquid to wash out the minute useful substance, the recovery rate of the minute useful substance can be increased to 80% or more.

### Reference Signs List

1, 2 Purifying and concentrating apparatus, 10 Raw material supply unit, 11 Container, 20 Removing unit, 21 Carrier column, 22 Filtration member, 23 First tank (roughly purified liquid storage tank), 24 Tank (pretreated liquid storage tank), 25 Sixth tank (cleaning liquid storage tank), 26 Seventh tank (equilibrating liquid storage tank), 30 Purifying and concentrating unit, 31 Second tank, 32 Third tank, 33 Hollow fiber membrane module, 33a First liquid inlet/outlet, 33b Second liquid inlet/outlet, 33c, 33d Third liquid inlet/outlet, 34 Pressurizing device, 35, 36 In-tank liquid amount monitoring device, 41 to 43 Flow path, 80 Fourth tank (diluent storage tank), 81 Fifth tank (recovery tank), 91 Fan filter device, 92 UV lamp, 110 First control unit

## Claims

1. A purifying and concentrating apparatus for purifying and concentrating a liquid containing a minute useful substance, the purifying and concentrating apparatus comprising:
a raw material supply unit configured to supply a liquid containing a minute useful substance;
a removing unit configured to remove an impurity from the liquid;
a purifying and concentrating unit configured to purify and concentrate, with a hollow fiber membrane, the liquid supplied from the raw material supply unit or the removing unit; and
a first control unit configured to control a timing of flow of the liquid from the raw material supply unit to the purifying and concentrating unit or flow of the liquid from the removing unit to the purifying and concentrating unit.

2. The purifying and concentrating apparatus according to claim 1, wherein the removing unit includes a carrier column configured to remove the impurity from the liquid, and the carrier column is connected to a flow path through which the liquid from which the impurity has been removed is supplied to the purifying and concentrating unit.

3. The purifying and concentrating apparatus according to claim 1 or 2, wherein
the removing unit includes a filtration member that removes the impurity from the liquid, and
the filtration member is interposed in a flow path through which the liquid is supplied from the raw material supply unit to the removing unit.

4. The purifying and concentrating apparatus according to claim 2, wherein
the removing unit includes a first tank that stores the liquid treated by the carrier column,
the first tank is disposed in a flow path through which the liquid is supplied from the carrier column to the purifying and concentrating unit, and the first tank is connected to a flow path through which the liquid in the first tank is supplied to the purifying and concentrating unit, and
the first control unit controls a timing at which the liquid in the first tank is supplied to the purifying and concentrating unit.

5. The purifying and concentrating apparatus according to claim 1 or 2, wherein the hollow fiber membranes have an average inner diameter of from 0.2 to 1.4 mm and a molecular weight cutoff of from 10000 to 1000000.

6. The purifying and concentrating apparatus according to claim 1, wherein
the purifying and concentrating unit includes a hollow fiber membrane module in which a plurality of the hollow fiber membranes are accommodated in a container having an inlet/outlet for the liquid, and
the container has at least a first liquid inlet/outlet and a second liquid inlet/outlet for supplying the liquid to the hollow fiber membranes, and a third liquid inlet/outlet for discharging the liquid that has permeated through the hollow fiber membranes.

7. The purifying and concentrating apparatus according to claim 6, wherein the hollow fiber membrane module is of an internal pressure type.

8. The purifying and concentrating apparatus according to claim 6, wherein the purifying and concentrating unit includes:
a second tank that stores the liquid supplied from the raw material supply unit or the removing unit, the second tank being connected to a flow path through which the liquid is supplied to the first liquid inlet/outlet of the hollow fiber membrane module;
a third tank that stores the liquid supplied from the raw material supply unit or the removing unit, the third tank being connected to a flow path through which the liquid is supplied to the second liquid inlet/outlet of the hollow fiber membrane module;
a pressurizing device configured to selectively pressurize either the second tank or the third tank; and
a second control unit configured to perform control in such a manner that the pressurizing device selectively pressurizes an inside of the second tank or an inside of the third tank to alternately supply the liquid in the second tank and the liquid in the third tank to the hollow fiber membrane module.

9. The purifying and concentrating apparatus according to claim 8, wherein the second control unit sets values for a liquid amount in the second tank and a liquid amount in the third tank, and causes the pressurizing device to alternately pressurize the second tank and the third tank in such a manner that the liquid amount in the second tank and the liquid amount in the third tank are the set values.

10. The purifying and concentrating apparatus according to claim 9, wherein
each of the second tank and the third tank includes an in-tank liquid amount monitoring device including an optical sensor at an inlet/outlet of the liquid at a tank bottom portion,
the in-tank liquid amount monitoring device is configured to, by the optical sensor, irradiate an inside of the inlet/outlet of the liquid with light to detect a transmittance of the light and monitor the liquid amount in the corresponding tank based on a change in the transmittance, and
the second control unit determines the liquid amount in the second tank and the liquid amount in the third tank by the in-tank liquid amount monitoring device to determine a tank to be pressurized by the pressurizing device.

11. The purifying and concentrating apparatus according to claim 9, wherein
the purifying and concentrating unit includes a camera that monitors a liquid level position in the second tank and a liquid level position in the third tank, and
the second control unit determines the tank to be pressurized by the pressurizing device based on information on the liquid level position in the second tank and the liquid level position in the third tank output from the camera.

12. The purifying and concentrating apparatus according to claim 8, wherein
the purifying and concentrating unit includes:
a fourth tank that stores a diluent, the fourth tank being connected to a flow path through which the diluent is supplied to the third liquid inlet/outlet of the hollow fiber membrane module; and
a third control unit configured to control a timing at which the diluent in the fourth tank is supplied to the third liquid inlet/outlet of the hollow fiber membrane module, and
the third control unit supplies the diluent in the fourth tank from the third liquid inlet/outlet of the hollow fiber membrane module in a case where a total of the liquid amount in the second tank and the liquid amount in the third tank is equal to or less than a value set by the second control unit.

13. The purifying and concentrating apparatus according to claim 8,
wherein
the purifying and concentrating unit includes:
a fifth tank connected to the first liquid inlet/outlet or the second liquid inlet/outlet of the hollow fiber membrane module by a flow path, the fifth tank recovering the liquid in the second tank and the liquid in the third tank; and
a fourth control unit configured to control recovery of the liquid in the second tank and the liquid in the third tank to the fifth tank.

14. The purifying and concentrating apparatus according to claim 13, wherein
each of the second tank and the third tank includes an in-tank liquid amount monitoring device including an optical sensor at an inlet/outlet of the liquid at a tank bottom portion, and
the in-tank liquid amount monitoring device is configured to, by the optical sensor, irradiate an inside of the inlet/outlet of the liquid with light to detect a transmittance of the light and monitor a liquid amount in the corresponding tank based on a change in the transmittance, and
the fourth control unit causes the pressurizing device to selectively pressurize the inside of the second tank or the inside of the third tank to recover the liquid in the second tank and the liquid in the third tank to the fifth tank, and determines a liquid amount in the second tank and a liquid amount in the third tank by the in-tank liquid amount monitoring device to stop the pressurizing device.

15. The purifying and concentrating apparatus according to claim 13, wherein
the purifying and concentrating unit includes a camera that monitors a liquid level position in the second tank and a liquid level position in the third tank, and
the fourth control unit causes the pressurizing device to selectively pressurize an inside of the second tank or the inside of the third tank to recover the liquid in the second tank and the liquid in the third tank to the fifth tank, and stops the pressurizing device based on information on the liquid level position in the second tank and the liquid level position in the third tank output from the camera.

16. The purifying and concentrating apparatus according to claim 1 or 2, further comprising a management unit,
wherein
the purifying and concentrating apparatus has a configuration in which elements including the raw material supply unit, the removing unit, and the purifying and concentrating unit are disposed in a housing, and
the management unit is configured to manage a degree of cleanliness in the housing, and
the management unit includes a fan filter device that supplies filtered air into the housing.

17. The purifying and concentrating apparatus according to claim 16, wherein the management unit further includes a UV lamp.

18. The purifying and concentrating apparatus according to claim 2, wherein the removing unit includes a sixth tank that stores a cleaning liquid for cleaning the carrier column, and a seventh tank that stores an equilibrating liquid for equilibrating the carrier column.

19. The purifying and concentrating apparatus according to claim 3, wherein the filtration member is disposed below the raw material supply unit, and the liquid is supplied from the raw material supply unit to the filtration member by an own weight of the liquid.

20. The purifying and concentrating apparatus according to claim 8, wherein side surfaces of the second tank and the third tank in a longitudinal direction thereof are arranged in parallel with a longitudinal direction of the hollow fiber membrane module.

21. The purifying and concentrating apparatus according to claim 20, wherein
the removing unit includes:
a carrier column that removes an impurity from the liquid, the carrier column being connected to a flow path through which the liquid from which the impurity has been removed is supplied to the purifying and concentrating unit; and
a first tank that stores the liquid treated by the carrier column, and
the second tank and the third tank are connected to the carrier column and the first tank by a flow path, and are disposed at positions above the carrier column and the first tank.

22. The purifying and concentrating apparatus according to claim 1 or 2, wherein the minute useful substance is selected from the group consisting of an extracellular vesicle, an antibody, a virus, a protein, an enzyme, and a nucleic acid.

23. A method for producing the liquid in which the minute useful substance is purified and concentrated using the purifying and concentrating apparatus described in claim 1 or 2, the method comprising
supplying a raw material liquid containing the minute useful substance to the purifying and concentrating apparatus to remove an impurity from the liquid; and/or
purifying and concentrating the liquid with a hollow fiber membrane.

24. The method for producing a purified and concentrated liquid of a minute useful substance according to claim 23, wherein the removing the impurity from the liquid includes adsorbing and removing the impurity from the liquid by a carrier column.

25. The method for producing a purified and concentrated liquid of a minute useful substance according to claim 23, wherein the purifying and concentrating the liquid with the hollow fiber membrane includes subjecting the liquid to alternating tangential flow filtration with a hollow fiber membrane module.

26. The method for producing a purified and concentrated liquid of a minute useful substance according to claim 23, wherein the purifying and concentrating the liquid with the hollow fiber membrane includes subjecting the liquid to alternating tangential flow filtration with a hollow fiber membrane module while recovering the minute useful substance deposited on a surface of the hollow fiber membrane.

27. The method for producing a purified and concentrated liquid of a minute useful substance according to claim 23,
wherein
the purifying and concentrating the liquid with the hollow fiber membrane includes supplying a diluent to the concentrated liquid to further purify and concentrate the liquid, and
the diluent is supplied to the liquid in a case where an amount (B) of the concentrated liquid relative to an amount (A) of the raw material liquid (B/A) is from 1/2 to 1/200.

28. The method for producing a purified and concentrated liquid of a minute useful substance according to claim 24, wherein the removing the impurity from the liquid by the carrier column and the purifying and concentrating the liquid with the hollow fiber membrane are performed in parallel.

29. The method for producing a purified and concentrated liquid of a minute useful substance according to claim 23, wherein the minute useful substance is at least one selected from the group consisting of an extracellular vesicle, an antibody, a virus, a protein, an enzyme, and a nucleic acid.

30. A purifying and concentrating apparatus for purifying and concentrating a liquid containing a minute useful substance, the purifying and concentrating apparatus comprising:
a raw material supply unit configured to supply a liquid containing a minute useful substance; and
a purifying and concentrating unit configured to purify and concentrate, with a hollow fiber membrane module, the liquid supplied from the raw material supply unit,
wherein
the hollow fiber membrane module has a first liquid inlet/outlet and a second liquid inlet/outlet for supplying the liquid to a hollow fiber membrane, and a third liquid inlet/outlet for discharging the liquid having permeated through the hollow fiber membrane,
the purifying and concentrating unit includes
a second tank that stores the liquid supplied from the raw material supply unit, the second tank being connected to a flow path through which the liquid is supplied to the first liquid inlet/outlet of the hollow fiber membrane module,
a third tank that stores the liquid supplied from the raw material supply unit, the third tank being connected to a flow path through which the liquid is supplied to the second liquid inlet/outlet of the hollow fiber membrane module,
a liquid feeder configured to selectively feed the liquid in the second tank or the liquid in the third tank to the hollow fiber membrane module,
a second control unit configured to control the liquid feeder in such a manner that the liquid in the second tank or the liquid in the third tank is selectively supplied to the hollow fiber membrane module, and
a monitoring device configured to monitor an amount of the liquid in the second tank and an amount of the liquid in the third tank,
the second control unit sets values for a liquid amount in the second tank and a liquid amount in the third tank, and performs control in such a manner that the liquid feeder selectively feeds the liquid from the second tank or the third tank to the hollow fiber membrane module in a case where the liquid amount in the second tank and the liquid amount in the third tank are the set values, and
the liquid amount in the second tank and the liquid amount in the third tank are determined based on information from the monitoring device.

31. The purifying and concentrating apparatus according to claim 30, wherein the hollow fiber membrane has an average inner diameter of 0.2 to 2.0 mm and a molecular weight cutoff of 5000 to 3000000.

32. The purifying and concentrating apparatus according to claim 30 or 31, wherein the monitoring device includes a camera that monitors a liquid level position in the second tank and a liquid level position in the third tank.

33. The purifying and concentrating apparatus according to claim 30 or 31, wherein the monitoring device includes optical sensors each provided at an inlet/outlet of the liquid at a bottom portion of each of the second tank and the third tank, and configured to irradiate an inside of the inlet/outlet of the liquid with light to detect a transmittance of the light to monitor an amount of the liquid in the corresponding tank based on a change in the transmittance.

34. The purifying and concentrating apparatus according to claim 30 or 31, wherein the hollow fiber membrane module is of an internal pressure type.

35. The purifying and concentrating apparatus according to claim 30,
wherein
the purifying and concentrating unit includes:
a fourth tank that stores a diluent, the fourth tank being connected to a flow path through which the diluent is supplied to the third liquid inlet/outlet of the hollow fiber membrane module; and
a third control unit configured to control a timing at which the diluent in the fourth tank is supplied to the third liquid inlet/outlet of the hollow fiber membrane module, and
the third control unit supplies the diluent in the fourth tank from the third liquid inlet/outlet of the hollow fiber membrane module in a case where a total of the liquid amount in the second tank and the liquid amount in the third tank is equal to or less than a value set by the second control unit.

36. The purifying and concentrating apparatus according to claim 30 or 31,
wherein
the purifying and concentrating unit includes:
a fifth tank connected to the first liquid inlet/outlet or the second liquid inlet/outlet of the hollow fiber membrane module by a flow path, the fifth tank recovering the liquid in the second tank and the liquid in the third tank; and
a fourth control unit configured to control recovery of the liquid to the fifth tank.

37. The purifying and concentrating apparatus according to claim 36, wherein
the liquid feeder includes a pressurizing device, and
the fourth control unit causes the pressurizing device to selectively pressurize an inside of the second tank or an inside of the third tank to recover the liquid in the second tank and the liquid in the third tank to the fifth tank, determines the liquid amount in the second tank and the liquid amount in the third tank by the monitoring device, and causes the pressurizing device to stop.

38. The purifying and concentrating apparatus according to claim 30 or 31, wherein the minute useful substance is selected from the group consisting of an electroactive polymer, a rare metal colloidal particle, a pigment, a dye, an inorganic or organic nanoparticle, a protein, a hormone, a cytokine, a proliferative factor, an angiogenic factor, a growth factor, an enzyme, an antibody, a plasma protein, a virus, an extracellular vesicle, a fermentum, a yeast, a cell, polysaccharides, and microalgae.

39. The purifying and concentrating apparatus according to any one of claims 1, 2, 30, and 31, wherein a biomolecule of a type different from the minute useful substance is attached to at least a part of the hollow fiber membrane.

40. The purifying and concentrating apparatus according to claim 12 or 35, wherein
the hollow fiber membrane module has at least two third liquid inlets/outlets, and
the third control unit supplies the diluent in the fourth tank from one of the at least two third liquid inlets/outlets of the hollow fiber membrane module in a state where flow of the liquid through the first liquid inlet/outlet and the second liquid inlet/outlet of the hollow fiber membrane module is stopped, and then supplies the diluent in the fourth tank from at least one of the at least two third liquid inlets/outlets of the hollow fiber membrane module in a state where the flow of the liquid through the first liquid inlet/outlet and the second liquid inlet/outlet is enabled.

41. A method for producing a purified and concentrated liquid of a minute useful substance, the method comprising:
removing an impurity from a raw material liquid containing a minute useful substance and the impurity; and then,
purifying and concentrating the liquid,
wherein
the purifying and concentrating the liquid includes purifying and concentrating the liquid using a hollow fiber membrane module, and in a process of the purifying and concentrating, a diluent is injected under pressure from a permeated liquid side of the hollow fiber membrane module to perform backwashing treatment on the hollow fiber membrane.

42. The method for producing a purified and concentrated liquid of a minute useful substance according to claim 41, wherein the hollow fiber membrane accommodated in the hollow fiber membrane module is made of a resin material containing cellulose acetate.

43. The method for producing a purified and concentrated liquid of a minute useful substance according to claim 41, wherein the hollow fiber membrane accommodated in the hollow fiber membrane module has a molecular weight cutoff of 100000 to 1000000.

44. The method for producing a purified and concentrated liquid of a minute useful substance according to claim 41 or 42, wherein the removing the impurity from the liquid includes adsorbing and removing the impurity from the liquid by a carrier column.

45. The method for producing a purified and concentrated liquid of a minute useful substance according to claim 41 or 42, wherein the purifying and concentrating the liquid using the hollow fiber membrane module includes subjecting the liquid to alternating tangential flow filtration.

46. The method for producing a purified and concentrated liquid of a minute useful substance according to claim 41 or 42, wherein the purifying and concentrating the liquid using the hollow fiber membrane module includes supplying a diluent to the purified and concentrated liquid to further purify and concentrate the liquid, and the diluent is supplied to the liquid in a case where an amount (B) of the purified and concentrated liquid relative to an amount (A) of the raw material liquid (B/A) is from 1/2 to 1/200.

47. The method for producing a purified and concentrated liquid of a minute useful substance according to claim 44, wherein the removing the impurity from the liquid by the carrier column and the purifying and concentrating the liquid using the hollow fiber membrane module are performed in parallel.

48. The method for producing a purified and concentrated liquid of a minute useful substance according to claim 41 or 42, wherein the minute useful substance is at least one selected from the group consisting of an extracellular vesicle, an antibody, a virus, a protein, an enzyme, and a nucleic acid.

49. The method for producing a purified and concentrated liquid of a minute useful substance according to claim 41 or 42, wherein a biomolecule of a type different from the minute useful substance is attached to at least a part of the hollow fiber membrane.

50. The method for producing a purified and concentrated liquid of a minute useful substance according to claim 41 or 42, further comprising flushing the hollow fiber membrane module prior to the backwashing treatment.
